# EUROPEAN PATENT APPLICATION

(11) **EP 0 523 289 A1**
(43) Date of publication of application: **20.01.1993**
(21) Application number: 91250193.9
(22) Date of filing: 16.07.1991
(51) Int. Cl.: C07D 319/08, C07C 225/36, C07C 205/48, C07C 50/36, C07C 50/38, C07H 15/252

(54) **Processes for producing doxorubicin, daunomycinone, and derivatives of doxorubicin**

(71) Applicant: The Board of Regents of the University of Nebraska, Lincoln Nebraska 68583 (US)
(72) Inventor: Wheeler, Desmond M.S., Lincoln, Nebraska 68502 (US)
(74) Representative: UEXKÜLL & STOLBERG

(57) **Abstract**

To produce doxorubicin and its analogues, methyl 3alpha, 5alpha-dihydroxy-5beta-(trimethylsilylethynyl)-2alpha-nitromethylcyclohexane-1beta-carboxylate acetonide is condensed with 1,4-dihydro-4,4,5-trimethoxy-1-oxonaphthalene in the presence of 1,8-diazabicyclo-[5.4.0]undec-7-ene in an aprotic solvent to produce 3-[(2beta-carbomethoxy-4beta-ethynyl-4alpha, 6alpha-(di-0-isopropylidenyl)cyclohexanyl-1-yl]-nitromethyl-4,4,5-trimethoxy-1-oxo-1,2,3,4-tetrahydronaphthalene, which is cyclized to produce 9beta-ethynyl-12-hydroxy-7alpha,9alpha-(di-0-isopropylidenyl)-6-nitro-4,5,5-trimethoxy-5,5a,6,6a,7,8,9,10,10a,11-decahydro-11-naphthacenone. The decahydro-11-naphthacenone is converted to 7alpha-9alpha,(di-0-isopropylidenyl)-4,5-dimethoxy-9beta-ethynyl-12-hydroxy-6-nitro-6,6a,7,8,9,10,10a,11-octahydro-11,-naphthacenone. The octahydro-11-naphthacenone is oxidized to 7alpha-9alpha, (di-0-isopropylidenyl)-9beta-ethynyl-11-hydroxy-4-methoxy-6-nitro-7,8,9,10,-tetrahydro-5,12-naphthacenedione which is converted to 6-desoxy-6-nitrodaunomycinone, daunomycinone and related 6-substituted analogues of daunomycinone.

## Description

This invention relates to a process for producing doxorubicin (adriamycin), and derivatives of doxorubicin, through a novel synthesis of daunomycinone and its derivaties. These mycinones can be coupled by known methods to daunosamine to produce doxorubicin and its corresponding derivatives.

It is known that doxorubicin is isolated from Streptomyces peucetius. A suitable process for this isolation is described by F. Arcamone, in "Duxorubicin Anticancer Antibiotics," (Academic Press, New York (1981). Moreover, synthetic routes to doxorubicin are known and these are summarized, for example, by F. Arcamone (loc. cit.), K. Krohn, Angew. Chem. Int. Ed. Fngl., volume 25 (1986), page 790 and Tetrahedron, volume 46 (1989), page 291 and T.P. Kelly, Tetrahedron Symposium-in-Print, volume 40 (1984), pages 4537-4793.

A representative prior art procedure for converting daunomycinone to adriamycin is that proposed by Arcamone et al., United States patent 3,803,124. The prior art methods of obtaining daunomycinone have the disadvantage of being expensive.

To reduce these disadvantages, a mono ketal of a 1,4-naphthoquinone is prepared as the precursor of the CD rings characterized in that once preparing a precursor for Ring A with C₆ and C₁₁ attached and the stereochemistry at C₇ and C₉ established and coupling it to CD and then completing ring B. Preferably, the mono ketal of 1,4-naphthoquinone is the 4-mono ketal of juglone methyl ether and the precursor for the A ring, with C₆ and C₁₁ attached and the stereochemistry at C₇ and C₉ established. It has the general structure: 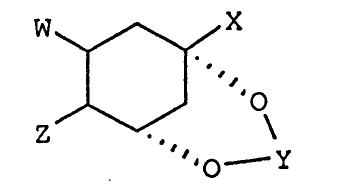
wherein W is an ester or similar group capable of acting as an electron pair acceptor in a Claisen condensation and related reactions, X is an umpolung equivalent of an acetyl group such as acetylide, trimethylsilylacetylide, ethyl or methyl vinyl ether, 2-methyl-1,3-dithiane or the corresponding dithioacetal or dithioacetal oxide, or cynaohydrin silyl ether or related compounds, Y is 2H, or if needed, a suitable protecting group for a 1,3 diol, such as a cyclic ketal e.g. acetonide or a cyclic ester e.g. boronate or carbonate, and Z is a one carbon umpolung of carbonyl such as nitromethyl or related compounds.

In one embodiment, daunomycinone and daunomycinones substituted at C₆ are produced by condensing the acetonide of methyl 3alpha,5alpha-dihydroxy-5beta-(trimethylsilylethynyl)-2alpha-nitromethylcyclohexane-1alpha-carboxylate with 1,4-dihydro-4,4,5-trimethoxy-1-oxonaphthalene in the presence of 1,8-diazabicyclo [5.4.0]undec-7-ene in an aprotic solvent to produce 3-[2beta-carbomethoxy-4beta-ethynyl-4alpha,6alpha-(di-O-isopropylidenyl)cyclohexan-1-yl]-nitromethyl-4,4,5-trimethoxy-1-oxo-1,2,3,4-tetrahydronaphthalene; and cyclizing said 1-oxotetrahydronaphthalene to produce 7alpha,9alpha-(di-O-isopropylidenyl)-9beta-ethynyl-12-hydroxy-6-nitro-4,5,5-trimethoxy-5,5a,6, 6a,7,8,9,10,10a,11-decahydro-11-naphthacenone. The decahydro-11-naphthacenone is converted to 7alpha,9alpha-(di-O-isopropylidenyl)-4,5-dimethoxy-9beta-ethynyl-12-hydroxy-6-nitro-6,6a,7,8,9,10,10a, 11-octahydro-11-naphthacenone, followed by oxidation of said decahydro-11-naphthacenone to 7alpha,9alpha-(di-O-isopropylidenyl)-9beta-ethynyl-11-hydroxy-4 methoxy-6-nitro-7,8,9,10-tetrahydro-5,12-naphthacenedione.

The 5,12-naphthacenedione is hydrolysed to 6-desoxy-6-nitrodaunomycinone, and reduced to 6-amino-7alpha,9alpha-ethynyl-11-hydroxy-4-methoxy-7,8,9,10-tetrahydro-5,12-napthacenedione. The aminonaphthacenedione is converted to the corresponding 6-diazoniumnaphthacenedione and from this obtaining: daunomycinone, 6-desoxydaunomycinone, the 6-desoxy-6-halodaunomycinones, 6-desoxy-6-cyanodaunomycinone and other 6-analogues easily derived from these products.

In this embodiment, said 1-oxotetrahydronaphthalene is cyclized with an alkali metal alkoxide of 1-6 carbon atoms and said decahydro-11-naphthacenone is converted to 7alpha,9alpha-(di-O-isopropylidenyl)-4,5-dimethoxy-9beta-ethynyl-12-hydroxy-6-nitro-6,6a,7,8,9,10,10a, 11-octahydro-11-napthacenone under acidic conditions.

The octahydro-11-naphthacenone is oxidized to 7alpha, 9alpha-(di-O-isopropylidenyl)-9beta-ethynyl-11-hydroxy-4-methoxy-6-nitro-7,8,9,10-tetrahydro-5,12-naphthacenedione with reagents such as cerium ammonium nitrate or thallium trinitrate or iodobenzenediacetate using with each oxidant a catalytic quantity of 2,3-dichloro-5,6-dicyanobenzoquinone.

In another embodiment, 11-substituted and 6,11-disubstituted daunomycinones are produced by condensing the acetonide of methyl 3alpha,5alpha-dihydroxy-5beta-(trimethylsilylethynyl)-2alpha-nitromethylcyclohexane-1alpha-carboxylate with 1,4-dihydro-4,4,5-trimethoxy-1-oxonaphthalene in the presence of 1,8-diazabicyclo [5.4.0]undec-7-ene in an aprotic solvent to produce 3-[2beta-carbomethoxy-4beta-ethynyl-4alpha,6alpha-(di-O-isopropylidenyl)cyclohexan-1-yl]-nitromethyl-4,4,5-trimethoxy-1-oxo-1,2,3,4-tetrahydronaphthalene; and cyclizing said 1-oxotetrahydronaphthalene to produce 7alpha,9alpha-(di-O-isopropylidenyl)-9beta-ethynyl-12-hydroxy-6-nitro-4,5,5-trimethoxy-5,5a,6, 6a,7,8,9,10,10a,11-decahydro-11-naphthacenone.

The decahydro-11-naphthacenone is converted to 7alpha,9alpha-(di-O-isopropylidenyl)-4,5-dimethoxy-9beta-ethynyl-12-hydroxy-6-nitro-6,6a,7,8,9,10,10a, 11-octahydro-11-naphthacenone followed by transforming said octahydro-11-naphthacenone to 7alpha,9alpha-(di-O-isopropylidenyl)11-imino-4-methoxy-6-nitro-6,6a,7,8,9,10,10a,11-octahydronaphthacene with methanol and ammonia and oxidizing the 7alpha, 9alpha-(di-O-isopropylidenyl)-9beta-ethynyl-11-imino-4-methoxy-6-nitro-6,6a,7,8,9,10,10a,11-octahydronaphthacene to 11-amino-7alpha,9alpha-(di-O-isopropylidenyl)-9beta-ethynyl-4-methoxy-6-nitro-7,8,9,10-tetrahydro-5,12-naphthacenedione with cerium ammonium nitrate and a catalytic amount of dichlorodicyanobenzoquinone or O₂/DMF (dimethylformamide).

The 11-amino-7alpha,9alpha-(di-O-isopropylidenyl)-9beta-ethynyl-4-methoxy-6-nitro-7,8,9,10-tetrahydro-5,12-napthacenedione is hydrolyzed to 11-amino-6,11-didesoxy-6-nitrodaunomycinone with mercuric oxide in acid and reduced with methanol and stannous chloride to 6,11-diamino-7alpha,9alpha-(di-O-isopropylidenyl)-9beta-ethynyl-4-methoxy-7,8,9,10-tetrahydro-5,12-naphthacenedione.

The 6,11-diamino-7alpha,9alpha-(di-O-isopropylidenyl)-9beta-ethynyl-4-methoxy-7,8,9,10-tetrahydro-5,12-naphthacenedione is converted to 6,11-diamino-6,11-didesoxydaunomycinone with HgO in acid.

Methyl cis-3,5-dihydroxy-5-(trimethylsilylethynyl)cyclohex-1-ene-1-carboxylate is synthesized from m-anisic acid by Birch reduction with lithium in ammonia to produce 5-oxocyclohex-2-ene-1-carboxylic acid, methylation of said cyclohex-2-ene-1-carboxylic acid to methyl 5-oxo-cyclohex-2-ene-1-carboxylate with diazomethane, trimethylsilylethynylation of said cyclohex-2-ene-1-carboxylate with cerium dichloride trimethylsilylacetylene to produce methyl cis-5-hydroxy-5-(trimethylsilylethynyl)cyclohex-2-ene-1-carboxylate, and conversion to an epoxide and opening of the resulting epoxide ring to give said cyclohex-1-ene-1-carboxylate.

The reaction mixture is kept cold during esterification with diazomethane and the ethanol that is present in the esterification reaction mixture is removed by azeotropic distillation with benzene. The methyl ester is treated with cerium dichloride trimethylsilylacetylene at a low temperature to prevent formation of a lactone, the epoxide is formed by oxidation with t-butyl hydroperoxide in the presence of molybdenum hexacarbonyl catalyst or by oxidation with cumyl hydroperoxide catalysed by titanium tetraisopropoxide and isolating the thus-produced epoxide and the epoxide ring is opened with 1, 8-diazabicyclo [5.4.0]-undec-7-ene or 1, 5-diazabicyelo [4.3.0]non-5-ene or sodium methoxide in methanol. The latter conditions also remove the trimethysilyl group to give methyl cis-3,5-dihydroxy-5-ethynylcyclohex-1-ene-1-carboxylate.

Methyl 3alpha,5alpha-dihydroxy-5beta-(trimethylsilylethynyl)-2alpha-nitromethylcyclohexane-1beta carboxylate is produced by reacting methyl cis-3,5-dihydroxy-5-(trimethylsilylethynyl)cyclohex-1-ene-1-carboxylate with excess of nitromethane in the presence of 1,8-diazabicyclo[5.4.0]undec-7-ene or 1,5-diazabicyclo[4.3.0]non-5-ene or sodium hydride in the presence of a substance which can form hydrogen bonds with hydroxyl groups.

Methyl 3alpha,5alpha-dihydroxy-5beta-ethynyl-2alpha-nitromethylcyclohexane-1beta-carboxylate is produced by reacting methyl cis-3,5-dihydroxy-5-beta-ethynylcyclohex-1-ene-1-carboxylate with nitromethane in the presence of 1,8-diazabicyclo[5.4.0]undec-7-ene or 1,5-diazabicyclo [4.3.0]non-5-ene or sodium hydride or potassium hydride in an aprotic solvent which can form hydrogen bonds with hydroxyl groups.

Methyl 3-alpha,5-alpha-dihydroxy-5-beta-(trimethylsilylethynyl)-2alpha-nitromethylcyclohexane-1-beta-carboxylate is produced by reacting methyl cis-(2,3alpha-epoxy-5alpha-hydroxy-5beta-(trimethylsilylethynyl)cyclohexane-1alpha-carboxylate with nitromethane in the presence of 1,8-diazabicyclo[5.4.0]undec-7-ene in an aprotic solvent.

As can be understood from the above description, the processes of this invention permit the manufacture of doxorubicin less expensively and permit the effective preparation of derivatives thereof.

The synthesis of the intermediate, required for elaboration of the A-ring, 3alpha,5alpha-dihydroxy-2alpha-nitromethyl-5beta-(trimethylsilylethynyl)-cyclohexane-1beta-carboxylate acetonide is shown in charts 1 and 2. It has been found that a 60 percent 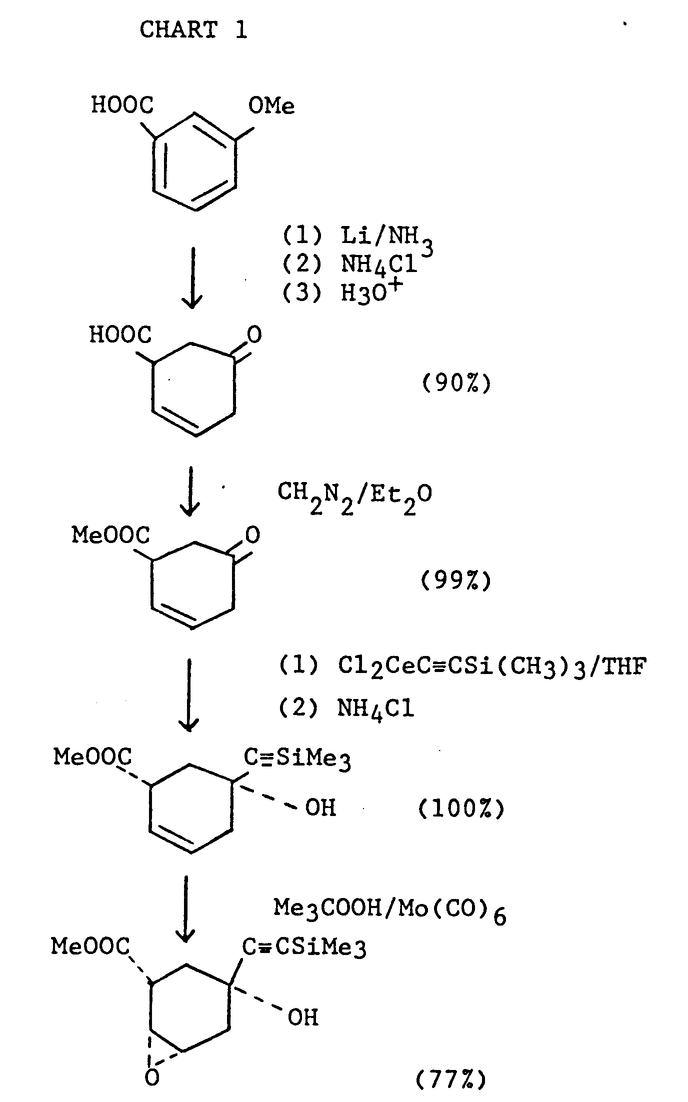 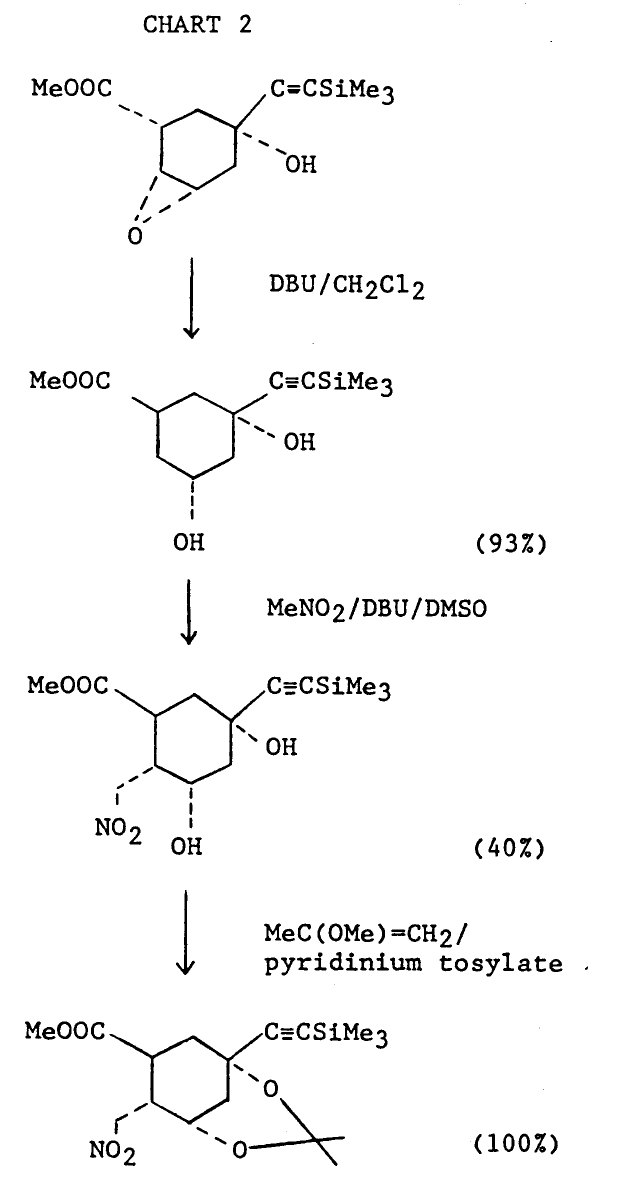
overall yield of methyl cis-3,5-dihydroxy-5-(trimethylsilylethynyl)cyclohex-1-ene-1-carboxylate can be obtained by keeping the reaction mixture cold during esterification with diazomethane and removing any ethanol and water present in the esterification reaction mixture by azeotropic distillation with benzene; quickly reacting to the thus-produced methyl ester with added cerium dichloride trimethylsilylacetylide at a low temperature to prevent formation of a lactone; forming the epoxide by oxidation with tert.-butyl hydroperoxide in refluxing benzene solution in the presence of molybdenum hexacarbonyl catalyst and isolating the thus-produced epoxide and opening the thus-formed epoxide ring with 1,8-diazabicyclo-[5.4.0]-undec-7-ene. Without the recited precautions and using completely anhydrous cerium dichloride, the yield from m-anisic acid is less than 10 percent.

Methyl 3alpha,5alpha-dihydroxy-2alpha-nitromethyl-5beta-(trimethylsilylethynyl)-cyclohexane-1beta-carboxylate was obtained from methyl cis-2,3-epoxy-5-hydroxy-5-(trimethylsilylethynyl) cyclohexane-1-carboxylate by reaction with nitromethane in the presence of 1,8-diazabicyclo-[5.4.0]-undec-7-ene in the presence of a hydrogen bond acceptor. Alternatively, methyl 3alpha,5alpha-dihydroxy-2alpha-nitromethyl-5beta-(trimethylsilylethynyl) cyclohexane-1beta-carboxylate was obtained from methyl cis-3,5-dihydroxy-5-(trimethylsilylethynyl)cyclohex-1-ene-1-carboxylate-and nitromethane in the presence of 1,8-diazabicyclo-[5.4.0]-undec-7-ene or sodium hydride in an aprotic solvent, that acts as a hydrogen bond acceptor.

The aprotic solvent used for the nitromethylation reaction may be selected from the following: hexamethylphosphoramide, dimethyl sulfoxide and NN'-dimethyl-NN'propylene urea or other hydrogen bond acceptors such as trimethyl-N-oxide or corresponding phosphorous or arsenic compounds. The preferred aprotic solvent is dimethyl sulfoxide. Nitromethane also adds to the 3,5-di-(terbutyldimethylsilyl) ether of the diol. The nitromethylation is conveniently carried out at ambient temperature for a time necessary to complete the reaction. The required time is of the order of 12 to 24 hours. If the reaction becomes exothermic, cooling to 15 to 25 degrees centigrade is preferred.

An acetonide is formed from 3alpha,5alpha-dihydroxy-5beta-(trimethylsilylethynyl)-2alpha- nitromethylcyclohexane-1beta-carboxylate by treatment with an acetonide-forming reagent. Representative acetonide-forming agents include, but are not limited to, 2-methoxypropene. Preferably, the acetonide-forming reagent is 2-methoxypropene.

The acetonide-forming reaction is carried out in the presence of a catalyst, such as pyridinium tosylate or p-toluenesulfonic acid. A preferred catalyst is pyridinium tosylate.

An inert solvent is used for the reaction. Benzene, heptane, toluene are representative of solvents suitable for the reaction. When benzene is used as the solvent, the reaction goes to completion within a few minutes' heating under reflux. Conditions appropriate for other solvents can be determined by routine experimentation.

The synthesis of a representative 1,4-dihydro-4,4,5-trimethoxy-1-oxo-naphthalene is shown in Chart 3. The initial steps of the synthesis include alkylation of both hydroxyl groups of 1,5-dihydroxynaphthalene, followed by selective removal of only one alkyl to give 5-alkoxy-1-naphthol. The alkyl moiety can be selected from primary alkyls of 1-6 carbon atoms, such as methyl, ethyl and n-propyl. The alkyl iodide is used in an amount in 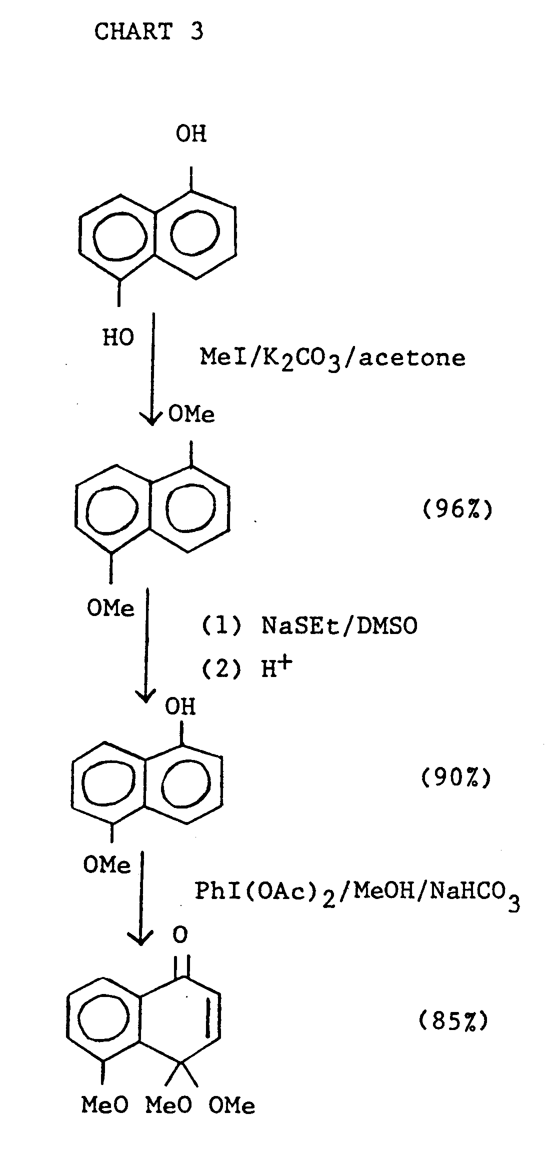
excess of that, required to alkylate both hydroxyl groups. Preferably, a molar ratio in the range of 4:1 to 2.5:1 of alkyl iodide to 1,5-dihydroxynaphthalene, is used. Most preferably, the ratio is about 3.5:1.

The etherification reaction is conducted in the presence of a base, which is normally selected from organic and inorganic bases. Representative bases include, but are not limited to, sodium carbonate, potassium carbonate, sodium hydroxide, potassium hydroxide, pyridine, triethylamine. A preferred base is potassium carbonate. The amount of base employed should be in the range of 3.5 moles to 4.5 moles, per mole of 1,5-dihydroxynaphthalene.

The aprotic solvent can be selected from acetone, methylethyl ketone, methylisobutyl ketone, tetrahydrofuran, acetonitrile, dimethyl sulfoxide. Preferably, the solvent is acetone.

The alkali metal alkanethiolate, used for removing one alkyl group from the intermediate dialkoxy compound can be selected from sodium and potassium alkanethiolates, having 1-6 carbon atoms in the alkane moiety. Preferably, the alkali metal alkanethiolate is sodium methanethiolate or ethanethiolate. The resulting alkali metal alkoxide can be acidified with any mineral acid, of which sulfuric acid, hydrochloric acid and phosphoric acid are representative.

The 5-alkoxy-1-napthol produced in this way can be converted, for example, to a corresponding juglone alkyl ether 4-ethylene ketal by reaction with ethylene glycol and thallium trinitrate or 1odobenzediacetate in trimethyl orthoformate. A preferred ketal for downstream synthesis is 1,4-dihydro-4,4-ethylenedioxy-5-methoxy-1-oxonaphthalene, produced from 5-methoxy-1-naphthol and ethylene glycol under the conditions stated.

A most preferred product for downstream synthesis is 1,4-dihydro-4,4,5-trimethoxy-1-oxonaphthalene. This is preferably produced from 5-methoxy-1-naphthol by reaction with iodobenzene diacetate in methanol with sodium bicarbonate. It will be understood that alkanols of 2-3 carbon atoms can be used to produce higher ketals by a similar process.

The synthesis of daunomycinone and 6-desoxy-6-nitrodaunomycinone from methyl 3alpha,5alpha-dihydroxy-2alpha-nitromethyl-5beta-(timethylsilylethynyl)cyclohexane-1-beta-carboxylate 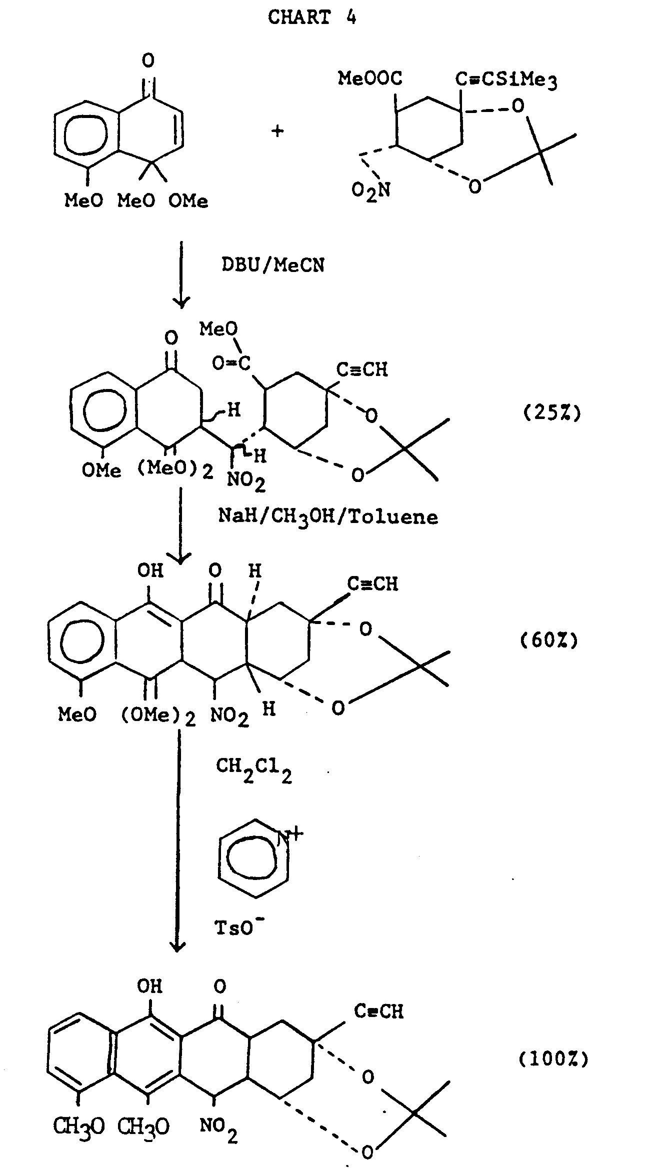 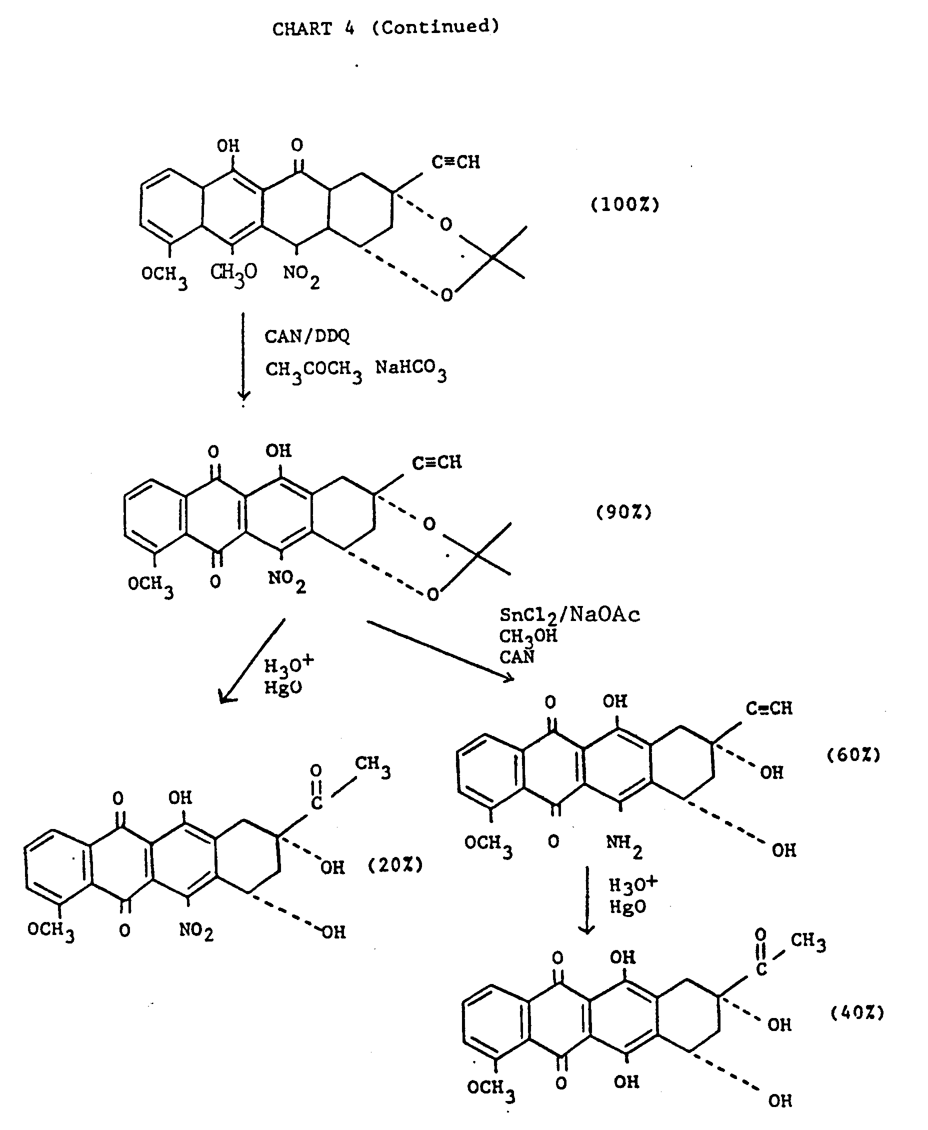
acetonide and a representative 1,4-dihydro-4,4-5-trimethoxyl-1-oxonaphthalene is shown in Chart 4. Condensation to the 1-oxotetrahydronaphthalene is carried out in an aprotic solvent. The aprotic solvent may be selected from dimethyl sulfoxide, hexamethylphosphoramide and acetonitrile. Acetonitrile is preferred. The reaction is carried out at ambient temperature. Conversion to a representative intermediate, 3-[(2beta-carbomethoxy-4beta-ethynyl-4alpha,6alpha-(di-O-isopropylidenyl)-cyclohexanyl-1-yl]-nitromethyl-4,4,5-trimethoxy-1-oxo-1,2,3,4-tetrahydronaphthalene is complete within 4-6 days.

The thus-produced 1-oxotetrahydronaphthalene is preferably cyclized with an alkali metal alkoxide of 1-6 carbon atoms. Suitable alkoxides can be prepared from alkanols of 1-6 carbon atoms, as above, and an alkali metal hydride. Alkali metal hydrides include sodium hydride or potassium hydride, of which sodium hydride is preferred. Most preferably, the alkali metal alkoxide is sodium methoxide. The cyclization is carried out in an inert solvent, such as benzene, hexane or toluene.

A representative cyclic product of this condensation is 7alpha,9alpha-(di-O-isopropylidenyl)-9beta-ethynyl-6-nitro-4,4,-5-trimethoxy- 5,5a, 6,6a,7,8,9,10,10a,10a,11-decahydro-11-naphthacenone.

The thus produced decahydro-11-naphthacenone loses methanol in the presence of an acidic catalyst to yield 7alpha,9alpha-(di-O-isopropylidenyl)-4,5-dimethoxy-9beta-ethynyl-12-hydroxy-6-nitro-6,6a,7,8, 9,10,10a,11-octahydro-11-naphthacenone. Possible catalysts include sulfonic acids e.g. methanesulfonic acid; pyridinium p-toluenesulfonate is preferred. A variety of aprotic solvents e.g. aromatic hydrocarbons, tetrahydrofuran, methylene chloride may be used; methylene chloride is preferred.

In one embodiment, the thus produced octahydro-11-naphthacenone was then oxidized to 7alpha,9alpha-(di-O-isopropylidenyl)9beta-ethynyl-11-hydroxy-4-methoxy-6-nitro-7,8,9,10-tetrahydro-5,12 naphthacenedione. Oxidizing agents include cerium ammonium nitrate (CAN) and thallium trinitrate (TTN); solvents include methanol, acetone and acetonitrile. The preferred conditions are CAN in acetone with dichlorodicyanobenzoquinone (DDQ) added in catalytic amount. The thus produced 5,12-naphthacenedione is then hydrolysed with acid in the presence of mercuric oxide to give 6-desoxy-6-nitrodauomycinone (chart 6).

Additionally, 6-nitro-5,12-naphthacenetrione (chart 5) was converted into 6-amino-7alpha,9alpha-(di-O-isopropylidenyl-9beta-ethynyl-12-hydroxy-7,8,9,10-tetrahydro-5,12-naphthacenetrione by reduction with SnCl₂H₂O in methanol in the presence of sodium on acetate at room temperature and the lenco product reoxidized with cerium ammonium nitrate. The amine compound has been converted into its diazonium compound with amyl nitrite in dimethoxyethane and then acid and finally mercuric oxide were added to form daunomycinone. A variety of other 6-desoxy-6-substituted daunomycinones are available from the diazonium compound. These mycinones can be coupled by known methods to daunosamine to produce doxorubicin and its corresponding derivatives. The sequence leading to these compounds is shown in chart 4.

In this embodiment, the aprotic solvent for condensing the acetonide with 1,4-dihydro-4,4,5-trimethoxy-1-oxonaphthalene is acetonitrile; the thus-produced 1-oxotetrahydronaphthalene is cyclized with sodium hydride in the presence of a catalytic quantity of methanol or toluene; the thus-produced 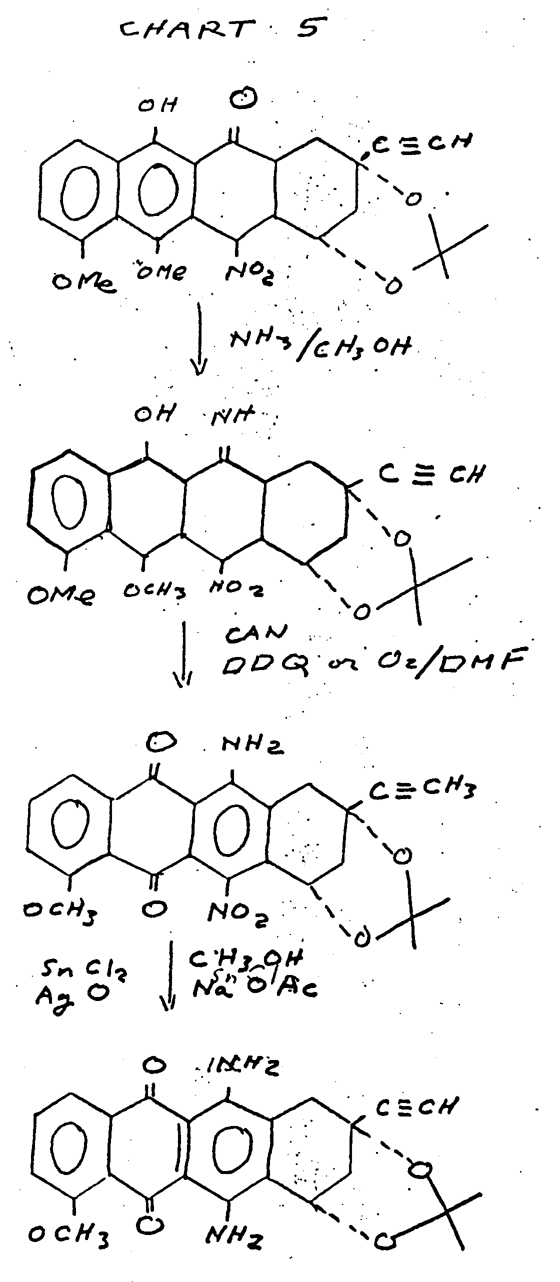 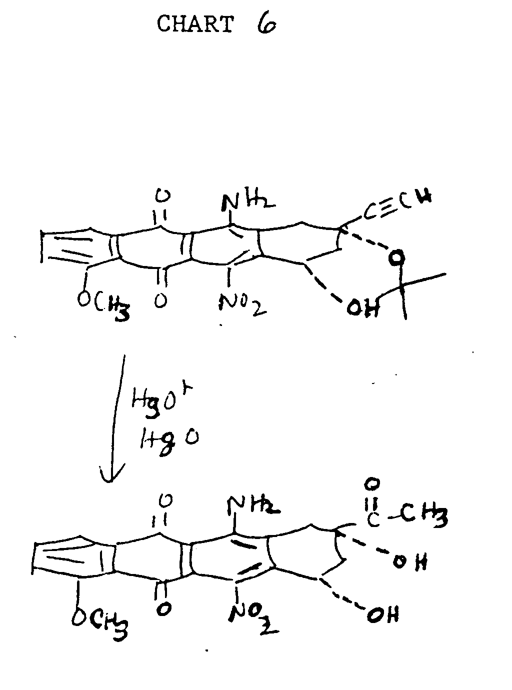
decahydro-11-naphthacenone is converted to the octahydro-11-naphthacenone in an acidic medium; the thus-produced octahydro-11-naphthacenone is oxidized to the tetrahydro-5,12-naphthacenedione with the thus-produced tetrahydro-5,12-naphthacenedione being converted to 6-desoxy-6-nitrodaunomycinone by hydrolysis and into daunomycinone and 6 to 8 daunomycinones in which the substituent at C₆ has been changed.

In a further embodiment, the aprotic solvent for condensing the acetonide with 1,4-dihydro-4,4,5-trimethoxy-1-oxonaphthalene is acetonitrile; the thus-produced 1-oxotetrahydronaphthalene is cyclized with sodium methoxide; the thus-produced decahydro-11-naphthacenone is converted to 9beta-acetyl-4,5-dimethoxy-6-nitro-7alpha,9alpha,12-trihydroxy-6,6a,7,8,9,10,10a,11-octahydro-11-napthacenone by hydrolysis with acid in the presence of mercuric oxide in an acidic medium; the thus-produced 9-beta-acetylocatahydro-11-naphthacenone is oxidized to 7alpha,9-alpha-dihydroxy-4-methoxy-6-nitro-6,6a,7,8,9,10,10a,11-octahydro-5,11,12-naphthacenetrione with thallium nitrate and the produced octahydro-5,11,12-naphthacenetrione is converted to daunomycinone.

In another embodiment, shown in chart 5, instead of oxidizing the octahydro-11-naphthacenone to 7alpha,9alpha-(di-O-isopropylidenyl)9beta-ethynyl-11-hydroxy-4-methoxy-6-nitro-7,8,9,10-tetrahydro-5,12-naphthacenedione, the nitrooctahydro-11-naphthacenone was treated with ammonia and 25 mL of methanol for 24 hours to yield 7alpha,9alpha-(di-O-isopropylidenyl)-9beta-ethynyl-12-hydroxy-11-imino-4-methoxy-6-nitro-6,6a,7,8,9,10,10a,11-octahydronaphthacene, which was oxidized with cerium ammonium nitrate in wet THF solution with sodium bicarbonate and a catalytic amount of dichlorodicyanobenzoquinone to yield 11-amino-7alpha,9alpha-(di-O-isopropylidenyl)-9beta-ethynyl-4-methoxy-6-nitro-7,8,9,10-tetrahydro-5,12-naphthacenedione needles in 89 percent yield. The same product was obtained in 90 percent yield by refluxing the nitroiminonaphthancene in O₂/DMF overnight. Chromatography was not needed.

To obtain 11-amino,6,11-didesoxy-6-nitrodaunomycinone (as shown in chart 7), the nitroaminonaphthacenedione in THF was hydrolysed with sulfuric acid and mercuric oxide.

Alternatively, the nitroaminotetrahydronaphthacenedione was reduced with stannous chloride in 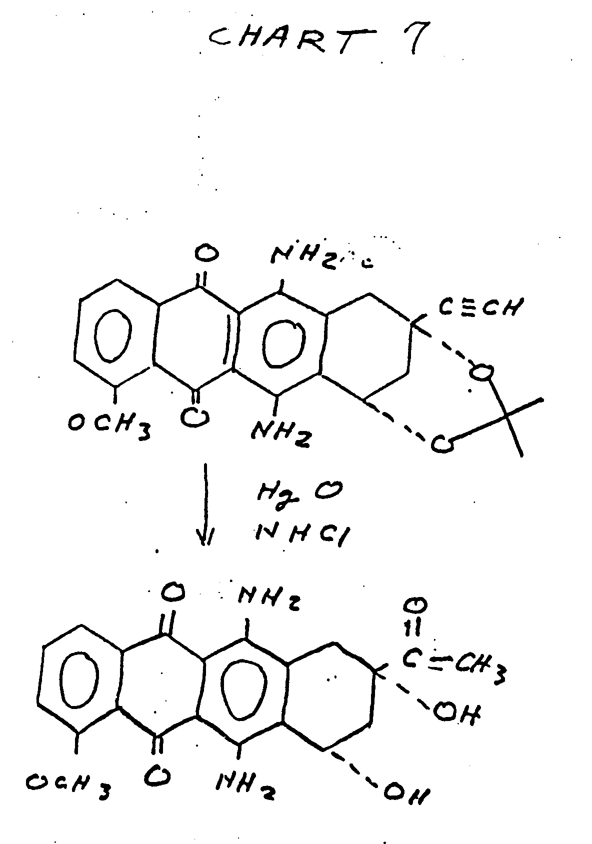
methanol with sodium acetate to produce 6,11-diamino-7alpha,9alpha-(di-O-isopropylidenyl)-9beta-ethyl-4-methoxy-7,8,9,10-tetrahydro-5,12-naphthacenedione which was then hydrolyzed with acid in the presence of mecuric oxide to 6,11-diamino-6,11-didesoxydaunomycinone (chart 5). These daunomycinone are the first derivatives with two non oxygen groups in ring B.

The amino group in the nitroaminodaunomycinone can be converted by the way of the 11-diazonium salt into halogens, cyano and other derivatives easily obtainable from these compouds, thus producing a series of 6-nitrodaunomycinones substituted at C₁₁. The 6-nitro group in these compounds can then be reduced to 6-amino and by way of the 6-diazonium compound into hydroxyl thus giving a series of daunomycinones substituted at C₁₁. Simarlarly, the 6-amino-11halo and cyano compounds can be converted through their 6-diazonium salts to a series of 6-halo or 6-cyano compounds. This leads a series of daunomycinones in which the hydroxyls at C₆ and C₁₁ have been replaced by halogens, cyano and other groups derived from these functionalities. The 6,11 diamino compound can be converted by way of the vis-diazonium compound into 6, 11-disubstituted derivatives (halogen, cyano and functionalities derived from these groups) in the substitutents at C₆ and C₁₁ are the same. Thus, the work makes available a large number of 6,11-daunomycinones which can be coupled with daunosamine to produce a new series of doxorubicins.

### EXAMPLES

Without further elaboration it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

In the following examples, the temperatures are set forth uncorrected in degrees centigrade. Unless otherwise indicated, all parts and percentages are by weight.

### Materials and Methods

¹H NMR spectra were recorded on a Varian EM-390 (90 MHx), a Varian VXR-200 (200MHx) or a Nicolet 1180 WB (360 MHz) spectrometer. Data are reported as chemical shifts in parts per million referenced to trimethylsilane internal standard (0 ppm) or to the residual chloroform peak of deuteriochloroform (7.26 ppm). Multiplicity, number of protons and coupling constants are reported. Couplings and/or coupling constants were determined using the COSY technique or by proton irradiation experiments.
¹³C NMR spectra were obtained on a Varian VXR-200 (50.4 MHz) spectrometer. Chemical shifts are referenced to the center peak of the residual chloroform triplet (77.0 ppm).
Ultraviolet spectra were obtained on a Hewlett-Packard 8450 UV-Vis spectrophotometer.
Infrared absorption spectra were recorded on a Perkin-Elmer Model 283 or an Analect RFX-30 spectrophotometer and are referenced to polystyrene (1601 cm⁻¹).
Elemental analyses were carried out by Desert Analytics (Tuscon, AZ).
Mass spectra were provided by the Midwest Center for Mass Spectrometry, University of Nebraska-Lincoln.
Melting points were determined in open-end capillary tubes on a Mel-Temp apparatus.
Column chromatography was carried out on E. Merck silica gel 60 (Flash, 40-230 mesh) and all thin layer chromatography (TLC) was carried out on commercial silica gel plates (Analtech Silica HLF 250 m or Merck Silica gel 60F 254).

High performance liquid chromatography (HPLC) was performed on a Waters Associates Model 600E chromatography apparatus, equipped with a Waters Associates Model R403 Differential Refractometer, using normal-phase silica gel columns.

Solvents were distilled before use over an appropriate drying agent under a nitrogen atmosphere. All reactions requiring anhydrous conditions were done under nitrogen in flame-dryed flasks. Solvents were evaporated using a Buchi rotary evaporator, then by evacuation at about 0.10 mm Hg at room temperature.

### Example 1

### 5-Oxocyclohex-2-ene-1-carboxylic Acid

The procedure of Biffin et al., Aust. J. Chem., vol. 25 (1972), page 1329, was used.

To a stirred slurry of 30 g (0.21 mol) of m-anisic acid (3-methoxybenzoic acid) in 300 mL of liquid ammonia were slowly added small pieces of lithium (3.5 g, 0.5 mol). Additional lithium (0.5 g, 0.07 mol) was added to maintain the blue color of the solution. The mixture was stirred for 2 hours and then cautiously quenched with solid ammonium chloride (45 g, 0.84 mol).

Ammonia was allowed to evaporate at room temperature. The last traces of ammonia were removed by warming the mixture under aspirator pressure. The resulting residue was dissolved in 200 mL of ice water and acidified to pH 1 with conc HCl. The resulting solution was extracted with four 100-mL portions of ethyl acetate. The combined ethyl acetate extracts were washed with one 50-mL portion of saturated sodium chloride solution, dried over sodium sulfate and evaporated to dryness. Crude semi-crystalline acid (24.1 g) was washed with a small amount of cold ether to yield 5-oxocyclohex-2-ene-1-carboxylic acid, off-white crystals, mp 98-101 degrees centigrade, lit (Biffin et al., supra, 98 to 99 degrees centigrade) yield 80 to 90 percent.
¹H NMR (CDCl₃): delta values 10.0 (s, 1H, COOH), 5.9 (m, 2H, HC=CH), 3.5 (m, 1H, CHCOO), 2.8 (m, 2H, C=CCH₂), 2.5 (m, 2H, CH₂).

### Example 2

### Methyl 5-Oxocyclohex-2-ene-1-carboxylate

A solution of 0.7M diazomethane in ether was slowly added to a solution of 6.0 g (42.8 mmol) of 5-oxocyclohex-2-ene-1-carboxylic acid in 100 mL of ether at 0 degrees centigrade, with swirling. The addition of diazomethane was continued until the bubbling ceased and a yellow color persisted. The solution was treated with a rapid stream of nitrogen until the yellow color was discharged. The solvent was removed by evaporation. Benzene (100 mL) was added and evaporated at room temperature using an aspirator. The residue was placed on a vacuum line for 2 hours to produce 6.5 g (42.4 mmol, 99 percent) of methyl 5-oxocyclohex-2-ene-1-carboxylate (oil).
¹N NMR (CDCl₃) : delta values 5.9 (m, 2H, HC=CH), 3.7 (s, 3H, COOMe), 3.5 (m, 1H, HCOO), 2.9 (m, 2H, C=CCH₂), 2.7 (m, 2H, CH₂).

### Example 3

### Methyl cis-5-Hydroxy-5-(trimethylsilylethynyl)-cyclohex-2-ene-1-1carboxylate

A solution of n-butyllithium (2.5 M, 16.8 mL, 42.1 mmol) was added dropwise, via a syringe, to a stirred solution of trimethylsilylacetylene (7 mL, 46.8 mmol) in 120 mL of tetrahydrofuran (THF) at -78 degrees centigrade. The resulting slurry was stirred at -78 degrees centigrade for 15 minutes and transferred via a cannula to slurry of 13.45 g (54.6 mmol) of cerium trichloride in 80 mL of THF at -78 degrees centigrade. The cerium chloride had been obtained by drying cerium trichloride heptahydrate (20.4 g, 54.6 mmol) at 140 to 160 degrees centigrade/0.1 mm Hg for 2 hours. Nitrogen was admitted to the cooled flask and 80 mL of dry THF was added to the resulting slurry, which was stirred overnight under nitrogen.

After the addition was complete (45 minutes), the resulting mixture was stirred for 1 hour at -78 degrees centigrade. The organocerium reagent, cerium dichloride trimethylsilylacetylide, was transferred over 45 minutes into a solution of 6.36 g (41.3 mmol) of methyl 5-oxocyclohex-2-ene-1-carboxylate in 80 mL of THF at -78 degrees centigrade. The resulting mixture was stirred for 1 hour more at -78 degrees centigrade and the reaction mixture was poured without warming into 400 mL of 10 percent ammonium chloride solution at 0 degrees centigrade. The resulting mixture was extracted with four 150-mL portions of ether. The combined organic extracts were washed with 200 mL of saturated aqueous sodium chloride solution and dried over sodium sulfate. Evaporation of the solvent yielded 10.04 g (41.1 mmol, 99.5 percent) of methyl cis-5-hydroxy-5-(trimethylsilylethynyl)cyclohex-2-ene-1-carboxylate, an oil.
IR(CH₂Cl₂): 3585 (COH), 2160 (C≡C), 1721 (COOCH₃), 1653 (C≡C) cm⁻¹.
¹NRM(CDCl₃): delta values 5.9-5.7 (m, 2H, CH=CN), 3.7 (s, 3H, COOCH₃), 3.3 (m, 1H, CHCOO), 2.6 (s, 1H, OH), 2.5 (dd, 1H, C=CCH₂), 2.4 (dd, 1H, C=CCH₂), 2.2 (m, 2H, CH₂), 0.2 (s, 9H, Si(CH₃)₃).
¹³C NMR (CDCl₃): delta values 174.0, 125.3, 123.4, 108.2, 87.4, 65.7, 51.9, 41.2, 39.1, 37.6.
MS, m/e (relative intensity): 252.21166 [M]⁺ (0.72), 237.0949 [M-CH₃]⁺ (29), 140.0657 (61). HRMS calcd for C₁₃H₂₀O₃Si: 252.1181.
Anal. Calcd for C₁₃H₂₀O₃Si: C, 61.86, H, 7.99. Found: C, 62.10, H, 8.13,

### Example 4

### Methyl cis-(2,3-Epoxy-5-hydroxy)-5-(trimethylsilylethynyl)cyclohexane-1-carboxylate

A solution of tert.-butyl hydroperoxide (4.1 M, 0.85 mL, 3.4 mmol) in isooctane was added to a stirred mixture of molybdenum hexacarbonyl (10 mg, 0.07 mmol) and 0.1753 g (0.6946 mmol) of methyl cis-5-hydroxy-5-(trimethylsilylethynyl)cyclohex-2-ene-1-carboxylate in 10 mL of benzene. The mixture was then heated under reflux for 2 hours. The mixture cooled and was washed with three 10-mL portions of water. The combined aqueous layers were extracted with three 5-mL portions of methylene chloride and dried over sodium sulfate. Evaporation of the solvent gave 0.1805 g (97 percent) of an oil. Flash chromatography of the oil (4:1 hexanes/ethyl acetate v/v) gave crystalline methyl cis-(2,3-epoxy-5-hydroxy)-5-(trimethylsilylethynyl)cyclohexane-1-carboxylate (0:131 g, 70 percent), mp. 74.1 to 74.6 degrees centigrade.
IR(KBr): 3360 (OH), 2160 (C≡C), 1732 (C=O), 1260, 845 cm⁻¹.
¹H NMR (CDCl₃): 3.8 (s, 3H, COOCH₃), 3.5 (m, 1H, CHCOO), 3.3 (m, 2H, HCOCH), 2.4 (dd, 1H, CH₂), 2.3 (s, 1H, OH), 2.0 (d, 1H, CH₂), 1.85 (m, 2H, CH₂), 0.17 (s, 9H, C≡CSi(CH₃)₃).
¹³C NMR (CDCl₃): 172.45 (COO), 107.43 (SiC≡C), 89.47 (C≡C), 66.26, 52.2, 51.80, 50.71, 40.92, 38.26, 31.03, 0.20 (Si(CH₃)₃).
MS m/r (relative intensity): 253.0897 (21.8) [M-CH₃]⁺, 236.0871 (38.2) [M-CH₃OH]⁺, 191.0035 (19.3), 159.0715 (16.6). Calcd for C₁₃H₂₀O₄Si: 253.0896.
Anal. Calcd for C₁₃H₂O₄Si: C, 58,18, H, 7.51. Found: C, 58.09, H, 7.65.

### Example 5

### Methyl cis-3,5-Dihydroxy-5-(trimethylsilylethynyl)cyclohex-1-ene-1-carboxylate.

A solution of 92.8 mg (0.345 mmol) of methyl cis-(2,3-epoxy-5-hydroxy)-5-(trimethylsilylethynyl)-cyclohexane-1-carboxylate and 52.5 mg (0.345 mmol) of 1,8-diazabicylo-[5.4.0]-undec-7-ene in 10 mL of methylene chloride was stirred for 2 hours at room temperature. The solvent was removed by evaporation and the residue was dissolved in a minimum amount of methylene chloride and filtered through a plug of flash silica gel (1:1 hexanes/ethyl acetate v/v). Evaporation of the solvent yielded methyl cis-3,5-dihydroxy-5-(trimethylsilylethynyl)cyclohex-1-ene-1-carboxylate, a colorless oil, which crystallized on standing, mp 89.8 to 90.8 degrees centigrade.
IR (CH₂Cl₂): 3310 (OH, 2158 (C≡C), 1721 (COOCH₃), 1653 cm⁻¹.
¹H NMR (CDCl₃): delta values 7.1 (s, 1H, C-CH), 4.4 (m, all couplings less than 2 Hz, 1H, CCH(OH)), 3.8 (s, 3H, COOCH₃), 3.0-3.4 (m, 1H, OH), 2.9-2.8 (dd, 2H, CH₂), 2.6 (s, 1H, OH), 2.5-2.2 (dd, 2H, CH₂), 0.17 (s, 9H, C-CSi(CH³)₃).
¹H NMR (D₆DMSO): delta values 7.1 (s, 1H, C=CH), 6.2 (2,1H, OH), 5.6 (d, 1H, OH), 4.7 (bs, 1H, CCH(OH)), 4.0 (s, 3H, COOCH₃), 3.0 (d, J = 17 Hz, 1H, C=CCH equatorial), 2.6 (d, J = 17 Hz, 1H, C=CCH axial), 2.5 (m, 1H, (HO)CCH axial), 1.9 (dd, J-10, 10 Hz, 1H (HO)CCH axial).
¹³C NMR (CDCl₃): 167.20 (C=O), 138.88, 127.60, 108.0, 88.52, 66.04, 61.62, 51.92, 41.74, 38.87, 0.228.
Anal. Calcd for C₁₃H₂₀O₄Si: C, 58.18; H, 7.51. Found: C, 58.24; H, 7.65.

### Example 6

### Addition of Nitromethane to Methyl cis-3,5-Dihydroxy-5-(trimethylsilylethynyl)cyclohex-1-ene-1-carboxylate

(i) Methyl cis-3,5-dihydroxy-5-trimethylsilylethynylcyclohex-1-ene-1-carboxylate (5.91 g, 22.0 mmol) was added in one portion to a mixture of nitromethane (12.2 g, 44.0 mmol) and DBU (6.7 g, 22.0 mmol) in 30 mL of dimethyl sulfoxide (DMSO). The mixture was stirred at room temperature for 15 hours and then neutralized to pH 6 with about 20 mL of 6 M HCl.
   The resulting mixture was extracted with four 100-mL portions of ethyl acetate. The organic phase was washed with 30 mL of saturated aqueous sodium chloride solution and dried over sodium sulfate. Evaporation of the solvent gave a crude oil product (10.0 g), which contained some DMSO.
   Flash chromatography in ethyl acetate/hexanes (3:7 v/v) gave methyl 3alpha,5alpha-dihydroxy-5beta-(trimethylsilylethynyl)-2alpha-nitromethylecyclohexane-1beta-1-carboxylate (trans isomer, 0. 8321g, 2.5 mmol, 11.5 percent); methyl 3alpha,5alpha-dihydroxy-5beta-trimethylsilylethynyl-2beta-nitromethylcyclohexane-2beta-carboxylate (the cis isomer, 1.95 g, 5.9 mmol, 26.9 percent); methyl 3alpha, 5alpha-dihydroxy-5beta-ethynyl-2alpha-nitromethylcyclohexane-1beta-carboxylate (trans desilylated isomer, 1.52 g. 5.9 mmol, 26.9 percent); methyl 3alpha,5alpha-dihydroxy-5beta-ethynyl-2beta-nitromethylcyclohexane-1beta-carboxylate (cis desilylated isomer, 1.28, 5.0 mmol, 22 percent). The overall yield of nitro adducts was 88 percent.
(ii) Nitromethane (8 mL, 112 mmol) was added to a slurry of sodium hydride (2.4 g, 56 mmol, 50 percent in mineral oil) in 50 mL of dry DMSO. The mixture was cooled as necessary to maintain an internal temperature of 20 degrees centigrade and stirred slowly for 1 hour. Methyl cis-3,5-dihydroxy-5-(trimethylsilylethynyl)cylohex-1-ene-1-carboxylate (3.0 g. 11.2 mmol) was added in one portion and the mixture was stirred for 24 hours at room temperature. To the resulting mixture was added 3 mL of conc HCl in ice. Additional HCl was added until the pH reached 7. The mixture was extracted with four 150-mL portions of ether, washed with 100 mL of saturated aqueous sodium chloride solution, dried over sodium sulfate and evaporated to give 3.39 g of residue, which contained some DMSO (TLC).
   Flash chromatography in ethyl acetate/hexanes (3:7 v/v) gave 2.12 g of product, characterized as a mixture of diastereomeric methyl 3,5-dihydroxy-5-(trimethylsilylethynyl)-2-nitromethylcyclohexane-1-beta-carboxylates.
   Trans isomer (1.021 g, 25.8 percent), methyl 3alpha, 5alpha-dihydroxy-5beta-(trimethylsilylethynyl)-2alpha-nitromethylcyclohexane-1beta- carboxylate:

IR (CH₂Cl₂): 3421, 2958, 2161, 1737, 1558 cm⁻¹.
¹H NMR (CDCl₃): delta values 4.7-4.6 (dd, 1H, CH₂NO₂), 4.0-4.1 (m, 1H, CHOH), 3.8 (bs, 2H, OH), 3.7 (s, 3H, COOCH₃), 2.8-2.9 (ddd, J = 4, 12, 12 Hz, CHCOOCH₃). 2.5-2.6 (m, 1H, CHCH₂NO2), 2.3-2.4 (m, 2H, CH₂ equatorial), 2.0 (dd, J = 12, 12 Hz, 1H, CH₂ axial), 1.9 (dd, J = 12, 3 Hz, 1H, CH₂ axial), 0.1 (s, 9H, Si(CH₃)₃).
¹³C NMR (CDCl ₃): delta values 173, 128, 106, 87, 75, 67.1, 66.8, 52, 41.5, 41.2, 36.7, 0.3.
Cis isomer (1.101 g, 19.8 percent), methyl 3alpha,5alpha-dihydroxy-5beta-(trimethylsilylethynyl)-2beta-nitromethylcyclohexane-1beta-carboxylate:
IR (CH₂Cl₂): 3423, 2956, 2160, 1739, 1556 cm⁻¹.
¹H NMR (CDCl₃): delta values 4.6 (dd, 1H, CH₂NO₂), 4.1 (m, 1H, CHOH), 3.7 (s, 3H, COOCH₃), 2.9 (bs, 2H, OH), 2.0-2.2 (mc, 4H, CH₂), 0.2 (s, 9H, Si(CH₃)₃).
¹³C NMR: delta values 173, 128, 106, 89, 74, 67.7, 67.1, 52, 41, 37, 30, 0.2.
Anal. Calcd for C₁₄H₂₂O₆NSi: C, 51.20, H. 6.75, N, 4.26. Found: C, 51.20; H, 7.19: N, 4.17.

### Example 7

### Addition of Nitromethane to Methyl cis-(2,3-Epoxy-5-hydroxy)-5-(trimethylsilylethynyl)cyclohexane-1-carboxylate

Nitromethane (4.2 g, 68 mmol) was added to a solution of 50 mL of DMSO and 5.2 g, (34 mmol) of DBU in 50 mL of DMSO. The mixture was stirred for 5 minutes, after which 1.8 g (6.8 mmol) of solid methyl cis-2,3-epoxy-5-hydroxy-5-(trimethylsilylethynyl)cyclohexane-1-carboxylate was added in one portion. The reaction was followed by TLC, using 1:2 v/v ethyl acetate/hexanes as elinant. After 2 hours, all of the epoxide had been consumed and a new spot, corresponding to diol appeared. After 16 hours, TLC showed that four new products and no starting materials or diol was present.

The reaction mixture was poured into 100 mL of cold 5 percent HCl solution and extracted with four 50-mL portions of ether and with 100-mL of saturated aqueous NaCl solution and dried over sodium sulfate. The ether was evaporated to yield 0.66 g of an oil, which was separated by flash chromatography (1:2 v/v ethyl acetate/hexanes) to yield methyl 3alpha, 5alpha-dihydroxy-5beta-(trimethylsilylethynyl)-2 alpha-nitromethylcyclohexane-1beta-carboxylate (0.28 g, 12.5 percent, trans nitro diol) and methyl 3alpha,5alpha-dihydroxy-5beta-(trimethylsilylethynyl)-2beta-nitromethylcyclohexane-1beta-carboxylate (0.31 g, 13.8 percent, cis nitro diol).

The aqueous phase was reextracted with methylene chloride and dried over sodium sulfate. Evaporation of solvent gave 0.52 g of an oil, separated by flash chromatography (1:1 v/v ethyl acetate/hexanes) into two desilylated nitroadducts, methyl 3alpha,5alpha-dihydroxy-5beta-ethynyl-2alpha-nitromethylcyclohexane-1beta-carboxylate (0.25 g, 12.4 percent, trans desilylated isomer) and methyl 3alpha, 5alpha-dihydroxy-5beta-ethynyl-2beta-nitromethylcyclohexane-2beta-carboxylate (0.19 g, 9.4 percent, cis desilylated isomer).

### Example 8

### Acetonide of Methyl 3alpha,5alpha-Dihydroxy-5 beta-(trimethylsilylethynyl)-2alpha-nitromethylcyclohexane-1beta-carboxyate

A mixture of 2-methoxypropene (260 microL, 3.6 mmol), trans nitro diol (methyl 3alpha,5alpha-dihydroxy-5beta-(trimethylsilylethynyl)-2alpha- nitromethylcyclohexane-1beta-carboxylate, 1.2 g, 3.6 mmol) and pyridinium tosylate (10 mg, 0.036 mmol) in 100 mL of benzene was stirred for at room temperature for 15 minutes. After 260 microL (3.6 mmol) of additional 2-methoxypropene was added, the resulting mixture was heated under reflux for 5 minutes. The resulting solution was cooled and washed with 30 mL of saturated aqueous sodium bicarbonate solution with 30 mL of saturated aqueous sodium chloride solution and dried over sodium sulfate. Evaporation of solvent yielded 1.22 g (3.22 mmol, 89 percent) of acetonide as an oil.
1R (neat ATR): 2990, 2164, 1735, 1556 cm⁻¹.
¹H NMR (CDCl₃): delta values 4.6-4.5 (dd, 1H, CHNO₂), 4.4-4.35 (dd, 1H, CHNO₂), 4.25-4.3 (m, 1H, CHOC), 3.7 (s, 3H, COOCH₃), 3.0 (ddd, J = 3, 4, 15 Hz, 1H, CH equatorial), 2.85 (ddd, J = 5, 12, 12 Hz, 1H, CHCOOCH₂), 2.6 (m, 1H, CHCH₂NO₂), 2.4 (ddd, J = 3, 5, 15 Hz, 1H, CH equatorial), 1.8 (m, 2H, CH axials), 1.6 (s, 3H, CH₃), 1.4 (s, 3H, CH₃), 0.16 (s, 9H, Si(CH₃)₃).
¹³C NMR (CDCl₃): delta values 173.6, 106.3, 97.9, 88.5, 75.2, 66.3, 65.4, 52.0, 41.8, 41.5, 38.7, 33.0, 31.4, 30.3, 0.3.
HRMS, m/e (relative intensity): 354.1366 [M-CH₃]⁺ (11.3), 294.1158 [M-OC₃ H₆]⁺ (5.4), 264.1184 [294-NO₂]⁺ (46.5), 73.0472 [TMSi]⁺ (100); calcd for C₁₆H₂₄NO₆Si 354.1373.

### Example 9

### Oxidation of 1-hydroxy-5-methoxy-naphthalene to 1,4-dihydro-4,4,5-trimethoxy-1-oxonaphthalene by Iodobenzene Diacetate

A solution of 15.5g (48 mmol) of iodobenzene diacetate and 22g of NaHCO₃ in 100 mL of methanol/trimethyl orthoformate (1/1) was stirred at room temperature for 30 minutes. To this mixture a solution of 3.74g of 1-hydroxy-5-methoxynaphthalene (21.5 mmol) in 250 mL of methanol was added dropwise. The color of the mixture changed from orange to greenish yellow over 2 hours at room temperature.

Ether (150 mL) was added to the mixture, and then sat. NaHCO₃ (75 mL). The aqueous washings were back extracted with ether (75 mL x 3). The combined ethereal extracts were washed with saturated aqueous sodium chloride solution and dried over sodium sulfate. The solution was evaporated to give 1,4-dihydro-4,4,5-trimethoxy-1-oxonaphthalene.

Flash chromatography of this product with elation using EtOAc:Hex 1:1 yielded 3.70g (74 percent) of crystals of 1,4-dihydro-4,4,5-trimethoxy-1-oxonaphthalene, mp 62-63 degrees centigrade.
¹H NMR (CDCl₃): delta values 7.7 (d, 1H, aromatic), 7.52 (t, 1H, aromatic), 7.16 (d, 1H, aromatic), 6.80 (m, 2H, HC=CH), 3.92 (s, 3H,OCH₃), 3.14 (s, 6H, (OCH₃)₂) (2 OCH₃).
UV (MeOH): maximum wavelength: 222 nm.
IR (neat): 1672, 1635, 1589, 1580, 1300, 1178, 1068, 1031 cm⁻¹.

### Example 10

### 3-[(2beta-Carbomethoxy-4beta-ethynyl-4alpha-6alpha-(di-O-isopropylidenyl)-cyclohexanyl-1-yl)]-nitromethyl-4,4,5-trimethoxy-1-oxo-1,2,3,4tetrahydronaphthalene

A solution of 0.904 g (3.0 mmol) of trans nitroacetonide (acetonide of methyl 3alpha, 5alpha-dihydroxy-5beta-(trimethylsilylethynyl)-2alpha-nitromethylcyclohexane-1beta-carboxylate), 1.34 g (5.7 mmol) of 1.4-dihydro-4,4,5-trimethoxy-1-oxonaphthalene and 750 microL (5 mmol) of DBU in 0.5 mL of acetonitrile was stirred for 5 days at room temperature. Methylene chloride (5 mL) was added to the mixture and the solution was chromatographed on a column packed with 150 g of silica gel (5 cm column, 30:70 v/v ethyl acetate/hexanes slurry). The material on the column was eluted with 400 mL of 30:70 v/v ethyl acetate/hexanes. After the nitro acetonide was eluted, as followed by TLC (iodine development), the chromatographic separation was continued with 1000 mL of 50:50 v/v ethyl acetate/hexanes.

Evaporation of the solvent from the various fractions gave recovered nitro acetonide (0.66 g, 2.2 mmol), recovered dimethyl ketal (1.03 g, 4.4 mmol) and product, 3-[(2beta-carbomethoxy-4beta-ethnynyl-4alpha,6alpha-(di-O-isopropylidenyl)-cyclohexanyl-1-yl)-nitromethyl]-4,4,5-trimethoxy-1-oxo-1,2,3,4-tetrahydronaphthalene (0.274 g, 0.51 mmol, 62 percent based on recovered acetonide) as an oily mixture of diasteromers.
IR (ATR thin film): 1733 (COOMe), 1686 (C=O), 1588, 1579 (NO₂) cm⁻¹.
¹H NMR (CDCl₃): delta values 7.6 (d, 1H, Ar), 7.4 (dd, 1H, Ar), 7.1 (d, 1H, Ar), 5.3-5.0 (d, 1H, CHNO₂), 4.4-4.0 (m, 1H, CHOC), 3.7 (s, 3H, ArOCH₃), 3.5 (s, 3H, COOCH₃), 3.3-2.8 (complex group of overlapping protons, 10H), including (COCH₃ ketal, 3.2, 3H, and 3.1, 3H), (O=CCH2, 2H), and (CHCOOCH₃, 1H), (HCCHNO₂, 1H)), 2.5 (s, 1H, C≡CH), 2.5 (d, 1H, CH₂), 2.2 (d, 1H, CH₂), 1.8 (d, 1H, NO₂CHCH), 1.6-1.5 (m, 2H, CH₂, 1.5 (s, 3H, CCH₃), 1.2 (s, 3H, CCH₃).
¹³C NMR (CDCl₃): mixture of two distereomers, (195.4, 195.1), (174.3, 173.2), (157.6, 157.5), (134.3, 134.0), (130.3, 130.2), (125.3), (120.05, 120.0), (117.2, 117.15), (101.6, 101.6), (97.9, 97.7), (86.1, 85.10), (84.48, 84.42), (72.6, 72.5), (66.0, 65.9) (64.5, 64.4), (55.9), (52.2, 51.6), (50.56, 50.46), (50.4, 50.1), (43.4 43.1), (42.8, 42.7), (42.0, 40.7), (39.77, 39.70), (35.8, 35.6), (32.7, 32.6), (31.2, 31.1), (30.05, 29.9).

### Example 11

### 9beta-Ethynyl-5,5-dimethoxy-12-hydroxy-7alpha, 9alpha-di-(O-isopropylidenyl)-4-methoxy-6-nitro-5, 5a,6,6a,7,8,9,10,10a-decahydro-11-naphthacenone

Sodium hydride (64 mg, 1.4 mmol) was added in one portion to a solution of 3-[(2beta-carbomethoxy-4beta-ethynyl-4alpha,6alpha-(di-O-isopropylidenyl)-cyclohexanyl-1-yl)]-nitromethyl-4,4, 5-trimethoxy-1-oxo-1,2,3,4-tetrahydronaphthalene (0.377 g, 0.7 mml) and methanol (80 drops) in 25 mL of toluene at 0 degrees centigrade. After 15 minutes' stirring, the cooling bath was removed. The reaction mixture was stirred for an additional 4 hours at room temperature and then washed briefly with 10 mL of cold 5 percent HCl. The organic phase was further washed with 5 mL of saturated aqueous sodium bicarbonate solution, with two 10-mL portions of saturated aqueous sodium chloride solution and dried over sodium sulfate. The solvent was evaporated to give 0.372 g of crude oily product, which was flash chromatographed (30:70 v/v ethyl acetate/hexanes, then 50:50 ethyl acetate/hexanes) to yield 0.212 g (0.42 mmol, 59.8 percent) of 9beta-ethynyl-5,5-dimethoxy-12-hydroxy-7alpha,9alpha-(di-O-isopropylidenyl)-4-methoxy-6-nitro-5,5a,6,6a,7,8,9,10,10a, 11-decahydro-11-naphthacenone, white crystalline solid, mp 234 degrees centigrade (decomp).
IR (ATR thin film): 3320 (C=C-OH), 2260 (C≡CH), 1559 (NO₂) cm⁻¹.
¹H NMR (CDCl₃): delta values 16.2 (s, 1H, C=COH), 7.8 (d, 1H, ArH), 7.6 (t, 1H, ArH) 7.1 (d, 1H, ArH), 5.2 (dd, J = 1, 3 Hz, 1H, CHNO₂), 4.6 (d, J = 4.5 Hz, 1H, CHOC), 3.9 (s, 3H, ArOCH₃), 3.9 (ddd, J = 4.5, 13, 13 Hz, 1H, O=CCH), 3.5 (d, J = 4.5 Hz, 1H, C≡C-CHCHNO₂), 3.4 (s, 3H, OCH₃ ketal), 3.1 (ddd, 1H, CH₂ equatorial), 3.0 (s, 3H, OCH₃ ketal), 2.9 (ddd, J = 3, 3, 13 Hz, 1H, CH₂ equatorial), 2.5 (s, 1H, C≡CH), 1.8 (d, J = 12 Hz, 1H, NO₂CHCHCO), 1.67 (d, 1H, CH₂ axial), 1.64 (s, 3H, OCCH₃), 1.5 (dd, J = 12, 12 Hz, 1H, CH₂ axial), 1.3 (s, 3H, OCCH₃).
¹³ C NMR (CDCl₃): HETCOR-DEPT 196.9 (C=O), 172.6 (ArC≧COH), 157.2 (ArC≡COH), 133.0 (Ar), 130.6 (ArH), 122.8 (Ar), 119.4 (ArH), 115.7 (ArH), 101.6, 101.3, 98.4, 85.2 (C≡CH), 79.9, 72.1 (CHNO₂), 69.5 (CHOC), 66.3, 55.8 (ArOCH₃), 52.5 (COCH₃), 51.4 (COCH₃), 46.0 (NO₂CCHCO), 44.8 (CHCNO₂), 40.2 (CH₂), 35.3 (CHC=O), 34.3 (CH₂), 30.8 (OCCH₃), 30.4 (OCCH₃).
UV-Vis: maximum wavelengths: 346, 341, 230 nm.

### Example 12

### 9beta-Acetyl-7alpha,9alpha,12-trihydroxy-4,5-dimethoxy-6-nitro-6,6a,7,8,9,10,10a,11-octahydro-11-naphthacenone

Sulfuric acid (1.5 mL, 3M) was added to a solution of 212 mg (0.42 mmol) of 9beta-ethynyl-5,5-dimethoxy-12-hydroxy-7alpha,9alpha-(di-O-isopropyl idenyl)-4-methoxy-6-nitro-5,5a,6,6a,7,8,9, 10,10a,11-decahydro-11-naphthacenone in 20 mL of THF. The solution immediately turned a bright yellow color. To the resulting solution was added 24.5 mg (0.11 mmol) of HgO. The resulting mixture was stirred for 4 hours at room temperature, cooled to 0 degrees centigrade and neutralized to pH 6 with 0.5 g of potassium hydroxide in 3 mL of water. The mixture was poured into 20 mL of water and extracted with four 20-mL portions of ethyl acetate. The combined organic layers were washed with two 20-mL portions of water and with 20 mL of saturated aqueous sodium chloride solution and dried over sodium sulfate. The solvent was removed by evaporation to yield 0.176 g (0.39 mmol, 93.2 percent) of 9beta-acetyl-7alpha,9alpha,12-trihydroxy-4,5-dimethoxy-6-nitro-6,6a,7,8,9,10,10a, 11-octahydro-11-naphthacenone, yellow solid. Product recrystallized from acetone gave fine yellow needle crystals, mp 186 degrees centigrade (decomp).
IR (ATR thin film) : 3427 (OH), 1711 (C=O), 1628, 1591, 1554 (NO₂) cm⁻¹.
¹H NMR (THF D₄): delta values 14.3 (s, 0.5H, ArOH), 8.0 (d, 1H, ArH), 7.5 (dd, 1H, ArH), 7.2 (d, 1H, ArH),m 6.4 (d, 1H, CHNO₂), 5.5 (bs, 0.5H, OH), 5.0 (bs, 0.5H, OH), 4.6 (m, 1H, CHOH), 3.9 (s, 2H, ArOCH₃ D-ring), 3.7 (s, 3H, ArOCH₃ C-ring), 3.7 (ddd, 1H, COCH), 2.6 (ddd, 1H, CH₂ equatorial), 2.5 (ddd, 1H, CH₂ equatorial), 2.3 (s, 3H, COCH₃), 1.8 (m, 1H, NO₂CHCH), 1.8-1.7 (m, 2H, CH₂ axials partly obscured by THF peak).
¹³C NMR (THF D₄): delta values 211.3 (C=O), 206.7 (C=O), 160.0, 157.3, 128.5, 122.4, 117.6, 117.5, 112.5, 112.4, 110.1, 81.9, 80.4, 69.87, 69.83, 62.7, 56.6, 46.0, 39.1, 36.2, 35.9, 35.8.
UV-Vis (MeOH) maximum wavelengths: 339, 321, 262, 220 nm.

### Example 13

### 9beta-Acetyl-7alpha,9alpha-dihydroxy-4-methoxy-6-nitro-5,6,6a,7,8,9,10,10a,11,12-decahydro-5,11-12-naphthacenetrione

Thallium nitrate (55 mg, 0.11 mmol) was added to a solution 50 mg (;0.11 mmol) of 9beta-acetyl-7 alpha,9alpha,12-trihydroxy-4,5-dimethoxy-6-nitro-6,6a,7,8,9,10,10a,11-octahydro-11-naphthacenone in 5 mL of acetone at 0 degrees centigrade. The mixture was brought to room temperature and stirred for 2 hours. Additional thallium trinitrate (11 mg, 0.02 mmol) was added and stirring was continued for 1 hour. The resulting solution was poured into 20 mL of water and extracted with three 25-mL portions of ethyl acetate. The combined organic extracts were washed with two 25mL portions of water and with 25 mL of saturated aqueous sodium chloride solution and dried over sodium sulfate. The solvent was evaporated to yield 99.5 mg of crude yellow product, an orange solid.

The product, which turned black upon standing, was dissolved in 5 mL of 9:1 ethyl acetate/hexanes v/v. After 16 hours, an orange precipitate had formed. The supernatant was removed by decantation and the resulting solids dried to give 25 mg (0.06 mmol, 54.5 percent) of 9beta-acetyl-7alpha,9alpha-dihydroxy-4-methoxy-6-nitro-5,6,6a,7,8,9,10,10a,11,-12-decahydro-5,11,12-naphthacenetrione, mp 242 degrees centigrade (decomp).
¹H NMR (acetone D₆): delta values 7.9 (dd, 1H, aromatic), 7.7 (d, 1H, aromatic), 7.6 (d, 1H, aromatic), 6.2 (d, J = 4.5 Hz, 1H, CHNO₂), 5.5 (s, 0.5, OH), 4.9 (m, 0.5, OH), 4.7 (m. 1H, CHOH), 4.0 (s, 3H, ArOCH₃), 3.5 (ddd, J = 4, 12, 13, Hz, 1H, COCHCH₂), 2.8 (ddd, J = 1, 5, 13 Hz, 1H, COCHCH₂ equatorial), 2.4 (ddd, 1H, CONCH₂ equatorial), 2.3 (s, 3H, COCH₃), 1.9 (m, 1H, NO₂ CHCH partially obscured by acetone quintet).
IR (ATR thin film): 1709 (C=O), 1663, 1665, 1587, 1560 (NO₂) cm⁻¹.
UV-Vis (MeOH) maximum wavelengths: 424 nm 274, 258 nm.

### EXAMPLE 14

### 7alpha,9alpha-(Di-O-isopropylidenyl)-4,5-dimethoxy-9beta-ethynyl-12-hydroxy-6-nitro-6,6a,7,8,9,10,10a, 11-octahydro-11-naphthacenone

A catalytic amount of pyridinium tosylate (5 mg) was added to a solution of 100 mg (0-20 mmol) of the decahydro-11-naphthacenone (example 11) in 20 mL of CH₂Cl₂. The mixture was refluxed for 6 hours while the color of the solution changed from light to shiny yellow. The reaction mixture was washed with water (2 x 20 mL) dried over Na₂SO₄ and the solvent was removed in vacuo. The yellow residue was recrystallized from hexane: ethyl acetate (9:1) to give the octahydro-11-naphthacenone as a mixture of 2 stereoisomers at C₆ (88 mg, yield 94 percent). The isomer can be separated by chromatography. Major isomer 85 percent m.p. 243-244 degrees centigrade.
IR (KBr) 3262, 1733, 1616, 1572, 1563, 1554, 1501, 1456, 1430, 1382, 1276, 1261, 1215 cm⁻¹.
NMR (360 MHz, CDCl₃): major isomer 14.01(s, 1H), 8.12(d, 1H), 7.49(t, 1H), 7.02(d, 1H), 6.35(d, J = 4 H₂1H), 4.73(d, 1H), 3.95(s, 3H), 3.27(m, 1H), 2.16(d, 1H), 1.61(s, 3H), 1.30(s, 3H), ppm.; (minor isomer 15 percent) 13.34(s, 1H), 8.05(d, 1H), 7.47(t, 1H), 7.05(d, 1H), 6.21(d, J = 10.4 H₂1H), 4.40(d, 1H), 3.91(s, 3H), 3.71(s, 3H), 3.66(d, 1H), 3.12(m, 2H), 2.53(s, 1H), 2.19(m, 2H), 1.67(s, 3H), 1.37(s, 3H) ppm.
UV Vis. (MeOH) maximum wavelength: 230, 266, 328, 396 nm.
Anal. Calcd. for C₂₅H₂₅O₈N: C, 64.2, H, 5.39, N, 3.00. Found: C, 64.9, H, 5.31, N: 3.04.

### EXAMPLE 15

### 7alpha-9alpha-(Di-O-isopropylidenyl)-9beta-ethynyl-11-hydroxy-4-methoxy-6-nitro-7,8,9,10-tetrahydro-5, 12-nathacenedione

A solution of 36 mg (0.075 mmol) of the octahydro-11-naphthacenone and 60 mg (0.675 mmol) of NaHCO₃ in 10 mL of acetone was treated with a solution of 167 mg (3mmol) of cerium ammonium nitrate (CAN) and 1 mg of dichlorodicyanobenzoquinone (DDQ) in 2 mL of acetone added in one portion at 0 degrees centigrade. The reaction mixture was stirred at room temperature for 1.5 hours during which the color changed from shiny yellow to orange red, and then 25 mL of CH₂Cl₂ was added. The mixture was filtered through over Celite. The organic layer was washed with saturated NaCl solution (2 x 20 mL), dried over Na₂^{SO}₄, and evaporated to give the tetrahydro-5,12-naphthacenedione as a red crystals (29 mg, 86 percent).mp greater than 258 degrees centigrade (decomp).
IR (KBr) 3306, 1620, 1588, 1529, 1491, 1369 cm⁻¹.
¹H NMR (360 MHz, CD₂Cl₂ 15.00(s, 1H), 7.19 (d, 1H) 7.57(t, 1H), 7.36(d, 1H), 4.50(m, 1H), 4.00(s, 3H), 3.12(m, 2H), 2.65(m, 2H), 2.57(s, 1H), 1.68(s, 3H), 1.37(s, 3H) ppm.
UV - Vis. (MeOH) maximum wavelength: 223, 240, 248, 358, 440 nm; (CHCl₃) 246, 370, 390, 416, 436 nm.

### Example 16

### 6-Desoxy-6-nitrodaunomycinone

A solution of 12 mg of (0.027 mmol) of the tetrahydro-5,12,-naphthacenedione in 2.5 mL of tetrahydrofuran was treated with 150 microL of H₂SO₄ (3M) and then 5 mg of mercuric oxide at 0 degrees centigrade and stirred at room temperature for 24 hours. The turbid solution was neutralized with NaHCO₃ to pH 8, and extracted with CH₂Cl₂ (10 x) 15 mL). The extract was dried over Na₂SO₄, and evaporated to yield 6-desoxy-6-nitrodaunomycinone as a red solid (7 mg, 61.4 percent). Flash chromatography on silica (60-200 mesh) eluting with 70:25:5(ethyl acetate:hexane:MeOH) afforded 2 mg of the pure mycinone as red crystals.
IR (CHCl₃), 3364, 1708, 1662, 1615, 1591, 1525, 1499, 1446, 1278, 1336 cm⁻¹.
¹H NMR (360) MHz, CD₂Cl₂ 15.15(s, 1H), 8.06 (d, 1H), 7.82(t, 1H), 7.42(d, 1H), 5.44(m, 1H, OH), 4.61(s, 1H), 4.09(s, 3H), 3.79(m, 1H, OH), 3.21(d, 1H), 3.00(d, 1H), 2.40(s, 3H), 2.30(d, 1H), 2.21(m, 1H) ppm.
UVVisi maximum wavelengths: (CHCl₃) 245, 250, 420, 446 nm; (MeOH) 360, 385, 418, 441, 466 nm.

### Example 17

### 6-Amino-9beta-ethynyl-4-methoxy-7alpha,9alpha,11-trihydroxy-7,8,9,10-tetrahydro-5,12-naphthacenedione

A solution of 15 mg (0.033 mmol) of the tetrahydro-5,12-naphthacenedione from example 15 and 96 mg (0.70 mmol) of NaOOCCH₃;3H₂O in 2 mL of CH₃OH was treated with SnCl₂H₂O (183 mg, 0.88 mmol), in 3 portions over a 90 min. at room temperature. During the addition the color of the solution changed to yellow. After the SnCl₂ was added, the mixture was stirred for 1 h, and the solvent was evaporated. A mixture of 15mL of CH₂Cl₂ and 15mL of saturated aqueous NaHCO₃ was added and the mixture was stirred for 30 min. During the stirring the color changed to pink and CO₂ was evolved.

The mixture was filled through Celite and extracted with CH₂Cl₂(3x20mL). The combined organic layers were washed with saturated aqueous NaCl, dried (Na₂SO₄), and evaporated to yield 14 mg of yellow solid. TLC showed that this was a mixture of 2 compounds. Flash chromatography using elution with hexane: ethyl aceate: MeOH(75:20:5) gave two compounds, the 6-desoxy-6-nitro starting material (6mg), and 6-amino-9beta-ethynyl-4-methoxy-5,7alpha,9alpha,11,12-pentahydroxy-7,8,9,10-tetrahy dronaphthacene (7.5mg) as shining yellow crystals (mp, 234-238 degrees centigrade).
NMR: 14.1(s, 1H,OH)O),13.4(s,2H,OH),8.1(d,1H), 7.7(t,1H),7.2(d,1H),5.0(m,1H),4.25(m,1H,OH),4.04(s, 3H),3.62(m,2H,NH₂),3.15(m1H,OH),2.95(q,2H), 2.55(s, 1H,C=CH),1.96(m,2H).ppm.
FT-IR (KBr) 3647, 3375,3289, 1671, 1634, 1601, 1582 cm⁻¹.
UV-Vis (CHCl₃) maximum wavelenghts 446,422,274,250 nm.

A saturated solution of 17.5 mg (0.032 mmol) ceric ammonium nitrate in acetone was added at O degrees centigrade to pentahydroxynaphthacene (7mg, 0.016 mmol) and NaHCO₃(10mg) in 2 mL of acetone. The reaction mixture was stirred for 5 h at room temperature and then filtered through Celite and extracted with CH₂Cl₂ (7x10mL). Evaporation yielded 7 mg of the amino trihydroxy-5,12-naphthacenone as purple red crystals.
¹H NMR (CD₂Cl₂), 14.80(s,1H,OH), 8.10 (d,1H), 7.85(t,1H),7.42(d,1H),5.35(d,1H),4.30(d,1H,OH), 4.09 (s,3H),3.57(m,2H),3.35(m,1H,OH),3.15(m,2H),2.55(s, 1H),1.95(m,2H)ppm.
UV-Vis (CHCL₃) maximum wavelength: 498, 442, 313, 269 nm.
FT-IR(KBr) 3644, 3277, 1671, 1631, 1580 cm⁻¹.

### Example 18

### Daunomycinone

A solution of 7 mg (0.018 mmol) of the amino-5,12-naphthacenedione and 1 mg of pyridinium tosylate in 2mL of dimethoxyethane was treated with 15 microL of isoamylnitrite. As the mixture was stirred at room temperature a purple precipitate formed, and 0.5mL of 0.01N HCL was added, after stirring had been continued for 5h, the visible spectrum of the mixture showed absorption at 515 nm. Then HgO (40 mg, 0.18mmol), was added and stirring was continued for 24 h. The mixture was filtered through Celite, extracted with CH₂Cl₂ (15x10mL) and evaporated to yield 10 mg of red solid. Flash chromatogrphy with elution with ethyl acetate:hexane:MeOH((90:5:5), followed by recrystalization from CHCl₃ gave 3mg of daunomycinone (mp 217-221 degrees centigrade).
¹H-NMR (CD₂Cl₂, 360 MHz), 14.22 (s, 1H), 13.47(2,1H), 8.21(d,1H), 8.00 (t,1H), 7.61 (d,1H), 5.48 (m,1H), 4.23 (s,3H), 4.07 (s,3H), 3.95 (d, 1H), 3.10 (m,2H), 2.57 (s,3H), 2.30 (m,2H) ppm.
FT-IR (CH₂Cl₂) 3540, 3053, 2927, 1712, 1616, 1578, 1420 cm⁻¹.
UV-Visi (MeOH) maximum wavelength: 532, 495, 476, 287, 253, 234nm.

### Example 19

### 7alpha,9alpha-(di-O-isopropylidenyl)-9beta-ethynyl-11-imino-4-methoxy-6-nitro-6,6a,7,8,9,10,10a,11-octahydronaphthacene

The nitrooctahydro-11-naphthacenone (10 mg, 0.021 mmol) was added to 25 mL of methanol which had been saturated with ammonia at 0 degrees C. The reaction mixture was stirred at room temperature with continued bubbling of NH₃ for 2 h (hours). The flow of ammonia was stopped; the reaction flask was sealed and stirring was continined at room temperature for 24 h. The color of the solution changed from yellow to brown. The reaction mixture was evaporated and the residine was flash chromatographed (60-200 mesh silica). Elution with ethyl acetate:hexane (25:75) gave 7alpha,9alpha-(di-O-isopropylidenyl)-9beta-ethynyl-12-hydroxy-11-imino-4-methoxy-6-nitro-6,6a,7,8,9,10,10a,11-octahydronaphthacene as yellow needles (9 mg, 0.018 mmol, 90 percent), m.p: 236-237 degrees C (210 degrees C, dec.); ¹H NMR (500 MHz, CDCl₃, ppm), 14.6 (b, 1H, Ar-OH), 8.2 (d, 1H, Ar-H), 7.4 (t, 1H, Ar-H), 7.1 (d, 1H, Ar-H) 6.9 (s, b, 1H, NH), 6.3 (d, J=3.2 Hz, 1H, H-CNO₂), 4.7 (d, J=4.5 Hz, 1H, H₇), 4.0 (m, 1H, H₁₀ₐ), 3.96 (s, 3H, OCH₃). 3.7 (s, 3H, OCH₃), 3.1 (dt, J_{gem} = 14.8 Hz, J_{H7-H8e} = 4.5 Hz, 1H, H₈ₑ), 2.7 (dt, J_{gem} = 14.8 Hz, J_{H7-H10e} = 12.8 Hz), 2.6 (s, 1H, C=CH), 2.0 (dd, J₆ₐ₋₁₀ₐ=12.3 Hz, J₆ₐ₋₆ = 3.2 Hz, 1H, H₆ₐ), 1.7 (q, 2H, H₁₀ₐₓ, H₈ₐ), 1.66 (s, 3H, CH₃), 1.3 (s, 3H, CH₃); ¹³C NMR (125MHz, CDCl₃, ppm), 176.3, 171.5, 156.0, 142,8, 134.9, 127.2, 122.5, 119.5, 118.5, 113.0, 103.3, 98.6, 84.9, 81.7, 72.7, 68.6, 66.0, 61.7, 56.5, 46.0, 40.8, 34.2, 30.6, 30.5; FT-IR (KBr neat, cm⁻ ¹), 3419, 3284, 2964, 2160, 1554, 1539, 1467; UV-Vis (CH₃OH, nm) 224 (15779), 273 (16300), 328 (3291), 428 (7854), 448 (7168); FAB-MS: 467 (M+1,45), 420 (100), 307 (15), 154 (100, 136 (85).

### Example 20

### 11-amino-7alpha,9alpha-(di-O-isopropylidenyl)-9beta-ethynyl-4-methoxy-6-nitro-7,8,9,10-tetrahydro-5,12-naphthacenedione

Cerium ammonium nitrate (18 mg, 0.035 mmol) and a catalytic amount of dichlorodicyanobenzoquinone (DDQ, 0.1 mg) and 20 mg of NaHCO₃ were added to the nitroiminonaphthacene (4 mg; 0.0086 mmol) in 1 mL wet THF solution. The reaction mixture was stirred at room temperature for four hours until TLC indicated that all of the starting material had reacted. The color of the solution had changed from deep yellow to red. The reaction mixture was filtered, and the solvent was evaporated. Flash chromatography of the residue involving elution with ethyl acetate-hexane (25:75) yielded 11-amino-7alpha,9alpha-(di-O-isopropylidenyl)-9beta-ethynyl-4-methoxy-6-nitro-7,8,9,10-tetrahydro-5,12-naphthacenedione (3.4 mg, 0.0076 mmol, 89 percent) reddish needles : m.p. 242-244 degrees C (dec.); 1H NMR (500 MHz, CDCl₃, ppm), 8.0 (d, 1H, Ar-H), 7.7 (t, 1H, Ar-H), 7.3 (d, 1H, Ar-H), 6.9 (b, 2H, NH₂), 5.0 (t, J=2.82 Hz, 1H, H-C-O), 4.0 (s, 3H, OCH₃), 3.1 (dd, 1H, J_{gem} = 16.9 Hz, H₁₀ₑ), 3.0 (dt, 1H, J_{gem} = 16.5 Hz, J₈ₑ₋₇ = 2.82Hz, H₈ₑ), 2.7 (d, 1H, H₁₀ₐ, J_{gem} = 16.9Hz), 2.65 (s, 1H C≡H), 2.1 (dd, 1H, J_{gem} = 16.9Hz, H₈ₐ), 1.6 (s, 3H, CH₃), 1.1 (s, 3H, CH₃); ¹³C NMR (125MHz, acetone-d₆, ppm), 181.0, 179.1, 148.3, 143.0, 136.5, 133.9, 133.7, 123.6, 118.5, 117.2, 116.4, 111.7, 98.1, 85.1, 71.6, 67.0, 64.2, 55.7, 39.4, 32.4, 30.4, 29.2, 28.9, 28.8; FT-IR (KBr neat, cm⁻¹), 3440, 3301, 2987, 2929, 2854, 2160, 1660, 1635, 1583, 1540, 1467; UV-Vis (CH₃OH, nm), 230 (11872), 372 (1314), 382 (1316), 474 (2666). The same product was obtained in 90 percent yield by refluxing the nitroiminonaphthancene in O₂/DMF overnight. Chromatography was not needed.

### Example 21

### 11-amino-6,11-didesoxy-6-nitrodaunomycinone

A solution of 10 mg (0.025 mmol) nitroaminonaphthacenedinoe in THF (2 mL) was treated with mercuric oxide (30 mg) and 1 mL of 1 N H₂SO₄. The reaction mixture was stirred at room termperature for 24 hours and then acidified to pH 1. The reaction mixture was extracted with ethyl acetate (3 x 20 mL). The combined organic layers were washed with saturated aqueous sodium chloride (30 mL) and dried over anhydrous sodium sulfate. The solvent was evaporated to give 10 mg crude product. Flash chromatography using 50 percent ethyl acetate and hexane as elutent gave 5 mg (55 percent) of 11-amino-6,11-didesoxy-6-nitrodaunomycinone as an orange solid; m.p. 274-275 degrees; ¹H NMR (500 MHz, DMSO-d₆, ppm), 7.92 (d, 1H, Ar-H), 7.85 (t, 1H, Ar-H), 7.60 (s, 2H, NH₂), 7.55 (d, 1H, Ar-H), 6.00 (s, 1H, OH), 5.60 (d, 1H, OH), 4.6 (m, 1H, H₇), 4.0 (s, 3H, OCH₃), 3.0 (d, 1H, H₁₀ₑ), 2.6 (d, 1H, K₁₀ₐ), 2.4 (dd, 1H, H₈ₑ), 2.35 (s, 3H, CH₃), 2.0 (dd, H, H₈ₐ); ¹³C NMR (125 MHz, dmso-D₆, ppm), 211.1, 183.7, 181.5, 159.6, 149.6, 145.4, 135.0, 132.8, 125.4, 120.8, 118.6, 117.7, 115.0, 110.0, 76.4, 65.8, 56.3, 40.0, 33.5, 23.9; FT-IR (KBr, cm⁻¹),, 3442, 3309, 1715, 1636, 1616, 1581, 1531; UV-Vis (CH₃OH, nm), 471, 430, 253, 221.

### Example 22

### 6,11-diamino-7alpha,9alpha-(di-O-isopropylidenyl)-9beta-ethynyl-4-methoxy-7,8,9,10-tetrahydro-5,12-naphthacenedione

A solution of 41 mg (0.091 mmol) of the nitroaminonaphthacenedione in methanol (5 mL) was treated with sodium acetate (120 mg, 0.9 mmol) and then stannous chloride (167 mg, 0.9 mmol) which was added in three portions over a period of 90 minutes at room temperature under a nitrogen atmosphere. After the mixture had been stirred overnight, its color had changed from orange to purple. Then 100 mL of CH₂Cl₂ and 10 mL of saturated aqueous sodium bicarbonate were added. The mixture was stirred at room temperature for one hour and filtered through Celite. The filtrate was washed with saturated aqueous sodium chloride (2 x 20 mL), and then dried over anhydrous sodiurn sulfate. The mixture was separated by chromatography (60-200 mesh silica treated with triethylamine and dried at 200 degrees C for 12 h). Elution with hexane:ethyl acetate (50:50) gave 11 mg of a pink solid and 28 mg of the starting compound. The pink solid was treated with silver oxide (148 mg 0.64 mmol) in 2 mL of ethanol for two hours. The solution was filtered and the filtrate was evaporated to yield a purple solid, purified by chromatography on the basic silica gel. Elution with hexane:ethyl acetate (50:50) yielded 6,11-diamino-7alpha,9alpha-(di-O)-isopropylidenyl)-9beta-ethyl-4-methoxy-7,8,9,10-tetrahydro-5,12-naphthacenedione, 8 mg (66 percent based on the recovered starting nitroaminonaphthacenedione) : mp:206-208 degrees C (dec); FT-IR (KBr, cm⁻¹), 3442, 3309, 3268, 1637, 1616, 1600, 1581; UV-Vis (CH₃OH, nm), 229 (17338), 249 (14415), 540 (4700); ¹H NMR (500 MHz, CDCl₃, ppm), 8.0 (d, 1Gm, Ar-H), 7.7 (t, 1H, Ar-H), 7.5 (d, 1H, Ar-H), 7.2 (s, 4H, NH₂), 5.0 (t, 1H, H₇), 4.0 (s, 3H, OCH₃), 3.5 (d, 1H, H₁₀ₑ), 2.9 (dt, 1H, H₈ₑ), 2.8 (d, 1H, H₁₀ₐ), 2.6 (s, 1H, C≡CH, h₈ₐ), 1.99 (d, 1H), 1.6 (s, 3H, CH₃), 1.1 (s, 3H, CH₃).

### Example 23

### 6,11-diamino-6,11-didesoxydaunomycinone

A solution of the diaminonaphthacendione (8 mg, 0.019 mmol) in 1 mL of THF was stirred at room temperature under a nitrogen atmosphere and 50 mg (0.23 mmol) of HgO and two drops of 2 N HCl were added. The reaction mixture was stirred at room temperature under nitrogen atmosphere overnight and filtered through Celite. The filtrate was evaporated and the residue was purified by chromatography on basic silica (60-200 mesh). Elution with ethyl acetate : hexane (70:30) gave 6,11-diaminodaunomycinone 3.5 mg (47 percent) as blue solid: ¹H NMR (500 MHz, CDCl₃, ppm), 8.1 (d, 1H, Ar-H), 7.8 (t, 1H, Ar-H), 7.4 (d, 1H, Ar-H), 7.2 (s, 4H, NH₂), 5.2 (t, 1H, H₇), 4.0 (s, 3H, OCH₃), 3.5 (b, 2H, OH), 3.2 (d, 1H, H₁₀ₑ), 2.9 (d, 1H, H₁₀ₐ), 2.4 (s, 3H, CH₃), 2.3 (d, 2H, H₈ₑ); FT-IR (KBr, cm⁻¹), 3417, 1710, 1652, 1617, 1602; UV-Vis (MeOH), 228, 249, 542.

The preceding examples can be repeated with similar success by substituting the generically or specifically described reactants and/or operating conditions of this invention for those used in the preceding examples.

Although preferred embodiments of the invention have been described with some particularity, many modifications and variations in the preferred embodiment are possible without deviating from the invention. Is it therefore to be understood that, within the scope of the appended claims, the invention may be practiced other than as specifically described.

## Claims

1. A process for the production of a compound of the general formula: 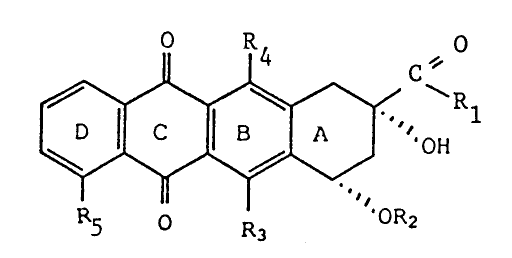 wherein R₁ is one of: CH₃ or CH₂CH;
R₂ is one of daunosamine, or H;
R₃ is one of -OH, NO₂, NH₂, F, Cl, Br, I, CN and H and groups derived from these; and
R₄ is one of OH, NO₂, NH₂, F, Cl, Br, I, CN and H or groups derived or substituted for these.
R₅ is OCH₃, OH or H;
in which:
a mono ketal of a 1,4-naphthoquinone is prepared as the precursor of the CD rings characterized in that once preparing a precursor for Ring A with C₆ and C₁₁ attached and the stereochemistry at C₇ and C₉ established and coupling it to CD and then completing ring B.

2. A process according to claim 1 characterized in that the mono ketal of 1,4-naphthoquinone is the 4-mono ketal of juglone methyl ether.

3. A process for the production of daunomycinone and related compounds in accordance with claim 1 characterized in that the precursor for the A ring, with C₆ and C₁₁ attached and the stereochemistry at C₇ and C₉ established, has the general structure: 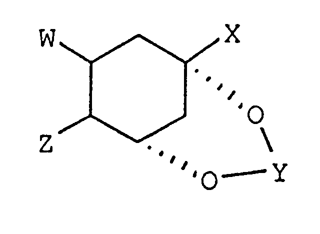 wherein W is an ester or similar group capable of acting as an electron pair acceptor in a Claisen condensation and related reactions;
X is an umpolung equivalent of an acetyl group such as acetylide, trimethylsilylacetylide, ethyl or methyl vinyl ether, 2-methyl-1,3-dithiane or corresponding dithioacetal or dithioacetal oxide, or cynaohydrin silyl ether or related compounds;
y is 2H, or if needed, a suitable protecting group for a 1,3 diol, such as a cyclic ketal e.g. acetonide or a cyclic ester e.g. boronate or carbonate;
Z is a one carbon umpolung of carbonyl such as nitromethyl.

4. A process for the production of daunomycinone, comprising condensing the acetonide of methyl 3alpha,5alpha-dihydroxy-5beta-(trimethylsilylethynyl)-2alpha-nitromethylcyclohexane-1alpha-carboxylate with 1,4-dihydro-4,4,5-trimethoxy-1-oxonaphthalene in the presence of 1,8-diazabicyclo [5.4.0]undec-7-ene in the presence of a hydrogen bond acceptor to produce 3-[2beta-carbomethoxy-4beta-ethynyl-4alpha,6alpha-(di-O-isopropylidenyl)cyclohexan-1-yl]-nitromethyl-4,4,5-trimethoxy-1-oxo-1,2,3,4-tetrahydronaphthalene; and cyclizing said 1-oxotetrahydronaphthalene to produce 7alpha,9alpha-(di-O-isopropylidenyl)-9beta-ethynyl-12-hydroxy-6-nitro-4,5,5-trimethoxy-5,5a,6, 6a,7,8,9,10,10a,11-decahydro-11-naphthacenone characterized by converting said decahydro-11-naphthacenone to 7alpha,9alpha-(di-O-isopropylidenyl)-4,5-dimethoxy-9beta-ethynyl-12-hydroxy-6-nitro-6,6a,7,8,9,10,10a, 11-octahydro-11-naphthacenone, oxidizing said octahydro-11-naphthacenone to 7alpha,9alpha-(di-O-isopropylidenyl)-9beta-ethynyl-11-hydroxy-4-methoxy-6-nitro-7,8,9,10-tetrahydro-5,12-naphthacenedione, hydrolysing said 5,12-naphthacenedione to 6-desoxy-6-nitrodaunomycinone, reducing said nitronaphthacenedione to 6-amino-7alpha,9alpha-(di-O-isoproylidenyl)-9beta-ethynyl-11-hydroxy-4-methoxy-7,8,9,10-tetrahydro-5,12-naphthacenedione, and converting said aminonaphthacenedione by way of the 6-diazonium derivatives to daunomycinone, 6-desoxydaunomycinone, the 6-desoxy-6-halodaunomycinones, 6-desoxy-6-cyanodaunomycinone and other 6-analogues easily derived from these products.

5. The process of claim 4 characterized in that said 1-oxotetrahydronaphthalene is cyclized with an alkali metal alkoxide of 1-6 carbon atoms.

6. The process of either claims 4 or 5 characterized in that said decahydro-11-naphthacenone is converted to 7alpha,9alpha-(di-O-isopropylidenyl)-4,5-dimethoxy-9beta-ethynyl-12- hydroxy-6-nitro 6,6a,7,8,9,10,10a, 11-octahydro-11-napthacenone under acidic conditions.

7. The process of any of claims 4-6 characterized in that said octahydro-11-naphthacenone is oxidized to 7alpha, 9alpha-(di-O-isopropylidenyl)-9beta-ethynyl-11-hydroxy-4-methoxy-6-nitro-7,8,9,10-tetrahydro-5,12-naphthacenedione with reagents such as cerium ammonium nitrate or thallium trinitrate or iodobenzenediacetate using with each oxidant a catalytic quantity of 2,3-dichloro-5,6-dicyanobenzoquinone.

8. A process for the production of daunomycinone, comprising condensing the acetonide of methyl 3alpha,5alpha-dihydroxy-5beta-(trimethylsilylethynyl)-2alpha-nitromethylcyclohexane-1alpha-carboxylate with 1,4-dihydro-4,4,5-trimethoxy-1-oxonaphthalene in the presence of 1,8-diazabicyclo [5.4.0]undec-7-ene in an aprotic solvent to produce 3-[2beta-carbomethoxy-4beta-ethynyl-4alpha,6alpha-(di-O-isopropylidenyl)cyclohexan-1-yl]-nitromethyl-4,4,5-trimethoxy-1-oxo-1,2,3,4-tetrahydronaphthalene; and cyclizing said 1-oxotetrahydronaphthalene to produce 7alpha,9alpha-(di-O-isopropylidenyl)-9beta-ethynyl-12-hydroxy-6-nitro-4,5,5-trimethoxy-5,5a,6, 6a,7,8,9,10,10a,11-decahydro-11-naphthacenone characterized by converting said decahydro-11-naphthacenone to 7alpha,9alpha-(di-O-isopropylidenyl)-4,5-dimethoxy-9beta-ethynyl-12-hydroxy-6-nitro-6,6a,7,8,9,10,10a, 11-octahydro-11-naphthacenone, converting said octahydro-11-naphthacenone to 7alpha,9alpha-(di-O-isopropylidenyl-11-imino-6-nitro-6,6a,7,8,9,10,10a,11-octahydronaphthacene with methanol and ammonia oxidizing the 7alpha, 9alpha-(di-O-isopropylidenyl)-9beta-ethynyl-11-imino-6-nitro-6,6a,7,8,9,10,10a,11-octahydronaphthacene to 11-amino-7alpha,9alpha-(di-O-isopropylidenyl)-9beta-ethynyl-4-methoxy-6-nitro-7,8,9,10-tetrahydro-5,12-naphthacenedione with cerium ammonium nitrate and a catalytic amount of dichlorodicyanobenzoquinone or O₂/DMF, hydrolyzing the 11-amino-7alpha,9alpha-(di-O-isopropylidenyl)-9beta-ethynyl-4-methoxy-6-nitro-7,8,9,10-tetrahydro-5,12-naphthacenedione to 11-amino-6,11-didesoxy-6-nitrodaunomycinone with acid and mercuric oxide, reducing said 11-amino-6-nitronaphthacenedione with stannous chloride in methanol to 6,11-diamino-7alpha,9alpha-(di-O-isopropylidenyl)-9beta-ethynyl-4-methoxy-7,8,9,10- tetrahydro-5,12-naphthacenedione.

9. A process according to claim 8 further characterized by converting the 6,11-diamino-7alpha,9alpha-(di-O-isopropylidenyl)-9beta-ethynyl-4-methoxy-7,8,9,10-tetrahydro-5,12-naphthacenedione to 6,11-diamino-6,11-didesoxydaunomycinone in HgO.

10. A process according to claim 8 characterized by converting the 11-amino-6,11-didesoxy-6-nitrodaunomycinone into 11-halo or 11-cyano derivatives and compounds obtained from them through the 11-diazonium-6-nitrodaunomycinone; or
converting the 11-halo or 11-cyano-6-nitrodaunomycinones to the corresponding 6-hydroxy-11-fluoro or 11-chloro or 11-bromo or 11-iodo or 11-cyano daunomycinones by way of the 6-diazonium compound; or
converting the said 6-diazonium compounds of the 11-halo and cyano compound to 6-fluoro or 6-chloro or 6-bromo or 6-iodo or 6-cyano daunomycinones; or
converting 6,11-diamino-6,11-didesoxydaunomycinone to 6,11-difluro or 6,11-dichloro or 6,11-dibromo or 6,11-diiodo or 6,11-dicyano daunomycinones.

11. A process of synthesizing methyl cis-3,5-dihydroxy-5-(trimethylsilylethynyl)cyclohex-1-ene-1-carboxylate from m-anisic acid characterized by Birch reduction with lithium in ammonia to produce 5-oxocyclohex-2-ene-1-carboxylic acid, methylation of said cyclohex-2-ene-1-carboxylic acid to methyl 5-oxo-cyclohex-2-ene-1-carboxylate with diazomethane, trimethylsilylethynylation of said cyclohex-2-ene-1-carboxylate with cerium dichloride trimethylsilylacetylene to produce methyl cis-5-hydroxy-5-(trimethylsilylethynyl)cyclohex-2-ene-1-carboxylate, and conversion to an epoxide and opening of the resulting epoxide ring to give said cyclohex-1-ene-1-carboxylate.

12. The process of claim 11 characterized in that the reaction mixture is kept cold during esterification with diazomethane and the ethanol that is present in the esterification reaction mixture is removed by azeotropic distillation with benzene, methyl ester is treated with cerium dichloride trimethylsilylacetylene at a low temperature to prevent formation of a lactone, the epoxide is formed by oxidation with t-butyl hydroperoxide in the presence of molybdenum hexacarbonyl catalyst or by oxidation with cumyl hydroperoxide catalysed by titanium tetraisopropoxide and isolating the thus-produced epoxide and the epoxide ring is opened with 1, 8-diazabicyclo [5.4.0]-undec-7-ene or 1, 5-diazabicyelo [4.3.0]non-5-ene or sodium methoxide in methanol; the latter conditions also remove the trimethysilyl group to give methyl cis3,5-dihydroxy-5-ethynylcyclohex-1-ene-1-carboxylate.

13. A process for producing methyl 3alpha,5alpha-dihydroxy-5beta-(trimethylsilylethynyl)-2alpha-nitromethylcyclohexane-1beta carboxylate characterized by reacting methyl cis-3,5-dihydroxy-5-(trimethylsilylethynyl)cyclohex-1-ene-1-carboxylate with excess of nitromethane in the presence of 1,8-diazabicyclo[5.4.0]undec-7-ene or 1,5-diazabicyclo[4.3.0]non-5-ene or sodium hydride in the presence of a substance that can form hydrogen bonds with hydroxyl groups.

14. A process for producing methyl 3alpha,5alpha-dihydroxy-5-beta-ethynyl-2alpha-nitro-methylcyclohexane-1beta-carboxylate comprising the steps of reacting methyl cis-3,5-dihydroxy-5-beta-ethynylcyclohex-1-ene-1-carboxylate with nitromethane in the presence of 1,8-diazabicyclo[5.4.0]undec-7ene or 1,5-diazabicyclo [4.3.0]non-5-ene or sodium hydride or potassium hydride in an aprotic solvent which can form hydrogen bonds with hydroxyl groups.

15. A process for preparing methyl 3-alpha,5-alpha-dihydroxy-5-beta-(trimethylsilylethynyl)-2alpha-nitromethylcyclohexane-1-beta-carboxylate characterized by reacting methyl cis-(2,3alpha-epoxy-5alpha-hydroxy-5beta-(trimethylsilylethynyl)cyclohexane-1alpha-carboxylate with nitromethane in the presence of 1,8-diazabicyclo[5.4.0]undec-7-ene in the presence of a substance that can form hydrogen bonds with hydroxyl groups.
